# EUROPEAN PATENT APPLICATION

(11) **EP 4 537 888 A1**
(43) Date of publication of application: **16.04.2025**
(21) Application number: 23202494.3
(22) Date of filing: 09.10.2023
(51) Int. Cl.: A61M 31/00, A61J 7/00

(54) **AN APPLICATOR FOR ARRANGING A MUCOADHESIVE SURFACE ON THE NASAL OR ORAL MUCOSA OF A PATIENT AND A DISPOSABLE PART FOR USE THEREWITH**

(71) Applicant: IQ medical GmbH, 82031 Grünwald (DE)
(72) Inventor: BEIER, Wolfgang, 81545 Munich (DE); HORSTKOTTE, Elke, 82061 Neuried (DE); MÜHLHÖLZL-ODÖRFER, Kathrin Ines, 80469 Munich (DE); SCHWEIZER, Adrian, 9500 Will SG (CH)
(74) Representative: Engelhard, Markus

(57) **Abstract**

The present invention relates to an applicator (100) for arranging a mucoadhesive surface, in particular a mucoadhesive film (204) loaded with an active agent such as a drug, on the nasal or oral mucosa, in particular the buccal mucosa, of a patient as well as to a disposable part (200) for use with an applicator for arranging a mucoadhesive surface on the nasal or oral mucosa of a patient and to a kit (10) comprising a disposable part having a mucoadhesive surface and an applicator for arranging said mucoadhesive surface on the nasal or oral mucosa of a patient.

## Description

### FIELD OF THE INVENTION

The present invention relates to an applicator for arranging a mucoadhesive surface, in particular a mucoadhesive film loaded with an active agent such as a drug, on the nasal or oral mucosa, in particular the buccal mucosa, of a patient as well as to a disposable part for use with an applicator for arranging a mucoadhesive surface on the nasal or oral mucosa of a patient and to a kit comprising a disposable part having a mucoadhesive surface and an applicator for arranging said mucoadhesive surface on the nasal or oral mucosa of a patient.

### BACKGROUND OF THE INVENTION

In recent years, the oral cavity as a site for local and systemic drug delivery has steadily gained importance. Drug-loaded mucoadhesive films, e.g., on polymer basis, can be arranged painlessly on the oral mucosa of a patient, in particular on the buccal mucosa, to directly release the drug (or any other active agent) into the oral tissue (e.g., by passive diffusion), which may allow for rapid delivery of the drug to the circulatory system. This route of application is not only well accepted by patients but additionally offers a number of pharmacological advantages such as fast onset of action, direct access to systemic blood and lymphatic circulation, avoidance of first-pass and food effects, elimination of degradation within the gastrointestinal tract prior to entering the systemic circulation, improvement in bioavailability and reduced drug exposure of the patient as well as easy access for self-medication with high convenience and flexibility.

However, the rate of release of the drug from the mucoadhesive film (e.g., the amount of drug released from the film in a certain time period) may depend on (a) the location of the mucoadhesive film on the mucosa, (b) the contact pressure of the mucoadhesive film on the mucosa, and (c) the degree of adhesion of the mucoadhesive film to the mucosa and/or the surface area of the mucoadhesive film adhering to the mucosa, all of which may affect the amount of wetting of the mucoadhesive film by saliva. In particular in case of self-medication, which naturally entails a wide range of possible errors in the application of the film, active cooperation and good compliance by the patient may thus be required to achieve controlled and reliable release of the drug from the mucoadhesive film and thus ultimately therapeutic success.

Therefore, there remains a need for means for arranging mucoadhesive films loaded with an active agent such as a drug on a mucosa of a patient that are easy to use and facilitate the controlled and reliable release of the active agent to the mucosa even in case of self-medication.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to an applicator for arranging a mucoadhesive surface, in particular a mucoadhesive film loaded with an active agent, on the nasal or oral mucosa, in particular the buccal mucosa, of a patient. The applicator comprises an insertable part and an exterior part connected to the insertable part, the insertable and exterior parts for insertion into the nasal or oral cavity of the patient and for placement on the skin of the patient, respectively, with the nasal or oral mucosa arranged therebetween. The insertable part comprises a retaining means that is configured to receive a disposable part comprising the mucoadhesive surface such that the mucoadhesive surface faces the nasal or oral mucosa when the nasal or oral mucosa is arranged between the insertable part and the exterior part. The applicator further comprises a means for pressing the mucoadhesive surface against the nasal or oral mucosa when the disposable part is received by the retaining means and the nasal or oral mucosa is arranged between the insertable part and the exterior part.

In some embodiments, the means for pressing the mucoadhesive surface against the nasal or oral mucosa is or comprises an elastic member, in particular a spring, configured to bias the disposable part, when received by the retaining means, or a part thereof towards the exterior part.

In some embodiments, the insertable part and/or the exterior part comprise(s) a different material than the elastic member.

In some embodiments, the elastic member and one or both of the insertable part and the exterior part are formed as a single piece, in particular by injection molding.

In some embodiments, the means for pressing the mucoadhesive surface against the nasal or oral mucosa is configured to press the mucoadhesive surface against the nasal or oral mucosa with a force of between 0.1 N and 50 N, preferably between 0.5 N and 10 N, most preferably between 2 N and 4 N.

In some embodiments, the applicator comprises a hinge that pivotably connects the insertable part and the exterior part.

In some embodiments, the insertable part and the exterior part each comprise a proximal handle portion, a central hinge portion and a distal clamping portion with the retaining means being arranged on and/or in the clamping portion of the insertable part, wherein the hinge connects the hinge portions of the insertable and exterior parts so as to allow for moving the clamping portions apart from each other by pushing the handle portions towards each other.

In some embodiments, the applicator comprises said elastic member and the hinge is formed by or comprises the elastic member or the elastic member extends between the handle portions of the insertable and exterior parts.

In some embodiments, the retaining means is or comprises a retaining structure that is configured to removably receive the disposable part, in particular via a snap fit or a press fit.

In some embodiments, the applicator further comprises a contact surface arranged between the insertable part and the exterior part, wherein the contact surface is to come in contact with an edge of the nasal or oral orifice of the patient, in particular a corner of the mouth, when the insertable part is inserted into the nasal or oral cavity of the patient to a predefined insertion depth to prevent further insertion of the insertable part beyond said predefined insertion depth, in particular wherein a distance between said contact surface and the retaining means is between 0.5 cm and 6 cm, preferably between 2 cm and 4 cm, most preferably between 2.5 cm and 3.5 cm.

In some embodiments, the insertable part comprises a disk-shaped portion in and/or on which the retaining means is arranged, wherein preferably a lateral physical dimension, in particular a diameter, of the disk-shaped portion is between 1.5 cm and 5 cm, most preferably between 2.5 cm and 4 cm.

In a second aspect, the present invention relates to a disposable part for use with an applicator for arranging a mucoadhesive surface on the nasal or oral mucosa of a patient, wherein the applicator comprises an insertable part and an exterior part connected to the insertable part, the insertable and exterior parts for insertion into the nasal or oral cavity of the patient and for placement on the skin of the patient, respectively, with the nasal or oral mucosa arranged therebetween. The disposable part comprises a carrier having a mucoadhesive surface and an attachment means for attaching the disposable part to the insertable part of the applicator such that the mucoadhesive surface of the disposable part faces the nasal or oral mucosa of the patient when the nasal or oral mucosa is arranged between the insertable part and the exterior part of the applicator.

In some embodiments, the mucoadhesive surface is arranged on a first side of the carrier and the attachment means is arranged on a second side of the carrier opposite to the first side.

In some embodiments, the disposable part is for use with an applicator according to the first aspect of the present invention in accordance with any one of embodiments described herein and the attachment means is configured to be received by the retaining means of the insertable part of the applicator.

In some embodiments, the attachment means is or comprises a mechanical connector that is configured to be removably received by the retaining means, in particular via a snap fit or a press fit.

In some embodiments, the mucoadhesive surface is formed by a mucoadhesive film that is loaded with an active agent, in particular a drug, and arranged on and/or in the carrier, in particular wherein said mucoadhesive film comprises a mucoadhesive polymeric matrix layer containing said active agent and, optionally, a backing layer that the mucoadhesive polymeric matrix layer is attached to.

In some embodiments, the active agent is or comprises a macrolide, in particular wherein the macrolide is selected from tacrolimus, sirolimus, everolimus, and pimecrolimus, preferably is selected from tacrolimus and sirolimus, more preferably is tacrolimus.

In some embodiments, the mucoadhesive surface is removably attached to the carrier, in particular by an adhesive, so as to allow for separating the mucoadhesive surface from the carrier when the mucoadhesive surface is arranged on the nasal or oral mucosa.

In a third aspect, the present invention relates to a kit comprising a disposable part having a mucoadhesive surface and an applicator for arranging said mucoadhesive surface on the nasal or oral mucosa of a patient. The applicator comprises an insertable part and an exterior part connected to the insertable part, the insertable and exterior parts for insertion into the nasal or oral cavity of the patient and for placement on the skin of the patient, respectively, with the nasal or oral mucosa arranged therebetween. The disposable part is attached or attachable to the insertable part of the applicator such that the mucoadhesive surface of the disposable part faces the nasal or oral mucosa when the nasal or oral mucosa is arranged between the insertable part and the exterior part of the applicator. The applicator comprises a means for pressing the mucoadhesive surface of the disposable part against the nasal or oral mucosa when the disposable part is attached to the insertable part of the applicator and the nasal or oral mucosa is arranged between the insertable part and the exterior part of the applicator.

In some embodiments, the applicator is an applicator according to the first aspect of the present invention in accordance with any one of the embodiments disclosed herein and/or the disposable part is a disposable part according to the second aspect of the present invention in accordance with any one of the embodiments disclosed herein.

In some embodiments, the means for pressing the mucoadhesive surface against the nasal or oral mucosa are configured to apply a pressure of between 0.1 kPa and 1000 kPa, in some examples between 0.5 kPa and 250 kPa, preferably between 2.5 kPa and 100 kPa, in some examples between 3 kPa and 60 kPa, most preferably between 4 kPa and 50 kPa, in one example between 5 kPa and 30 kPa and in one example between 10 kPa and 20 kPa, between the mucoadhesive surface of said disposable part and the nasal or oral mucosa.

In some embodiments, the mucoadhesive surface is formed by a mucoadhesive film removably attached to the disposable part so as to allow for separating the mucoadhesive film from the disposable part when the mucoadhesive film is arranged on the nasal or oral mucosa, in particular wherein the mucoadhesive film is removably attached to the disposable part by an adhesive, preferably an adhesive layer, having a pull-off force that is lower than a pull-off force for separating the mucoadhesive film from the nasal or oral mucosa after the mucoadhesive film was pressed against the nasal or oral mucosa by said means for pressing the mucoadhesive surface of the disposable part against the nasal or oral mucosa.

In a fourth aspect, the present invention relates to a method for arranging a mucoadhesive surface, in particular a mucoadhesive film loaded with an active agent, on the nasal or oral mucosa, in particular the buccal mucosa, of a patient using a disposable part comprising said mucoadhesive surface and an applicator for arranging said mucoadhesive surface on the nasal or oral mucosa of a patient. The applicator comprises an insertable part and an exterior part connected to the insertable part, wherein the disposable part is attached to the insertable part of the applicator. The method comprises inserting the insertable part of the applicator with the disposable part attached thereto into the nasal or oral cavity of the patient and placing the exterior part on the skin of the patient such that the nasal or oral mucosa is arranged between the interior and exterior parts with the mucoadhesive surface of the disposable part facing the nasal or oral mucosa. The method further comprises pressing the mucoadhesive surface of the disposable part against the nasal or oral mucosa using a means of the applicator for pressing the mucoadhesive surface of the disposable part against the nasal or oral mucosa.

In some embodiments, the applicator is an applicator according to the first aspect of the present invention in accordance with any one of the embodiments disclosed herein, the disposable part is a disposable part according to the second aspect of the present invention in accordance with any one of the embodiments disclosed herein, and/or the applicator and the disposable part are the applicator and the disposable part, respectively, of a kit according to the third aspect of the present invention in accordance with any one of the embodiments disclosed herein.

In some embodiments, the method further comprises attaching the disposable part to the applicator prior to inserting the insertable part of the applicator into the nasal or oral cavity of the patient.

In some embodiments, the mucoadhesive surface is formed by a mucoadhesive film removably attached to the disposable part and the method further comprises, after pressing the mucoadhesive surface of the disposable part against the nasal or oral mucosa, separating the mucoadhesive film from the disposable part while the mucoadhesive film remains arranged on the nasal or oral mucosa.

### DETAILED DESCRIPTION

The present invention aims at, for example, providing means for arranging mucoadhesive films loaded with an active agent such as a drug on a mucosa of a patient that are easy to use and facilitate the controlled and reliable release of the active agent to the mucosa, in particular in case of self-medication. For this, the present invention aims at, for example, facilitating arranging the mucoadhesive film at a defined position or application site on the mucosa. The present invention further aims at, for example, providing improved control of a contact pressure between the mucoadhesive film and the mucosa and of the degree of adhesion of the mucoadhesive film to the mucosa. The present invention aims at, for example, controlling and/or normalizing the application force or pressure of the mucoadhesive film across various subjects and/or anatomic sites. The present invention also aims at, for example, preventing use errors, in particular patient-specific sources of error in the application and positioning of the mucoadhesive film. It furthermore is an aim of the present invention to improve hygiene during application of the mucoadhesive film. It is another aim of the present invention to improve sustainability by minimizing the environmental footprint.

The present invention provides a disposable part having a mucoadhesive surface, for example a mucoadhesive film loaded with an active agent, and an applicator for arranging said mucoadhesive surface on the nasal or oral mucosa of a patient, for example on the buccal mucosa. The disposable part and the applicator may be provided together as a kit or may be provided separately as independent devices or units. In the following, various features of the disposable part and the applicator will be described. Unless the context indicates otherwise, each of these features or any combination thereof may be present in the applicator according to the first aspect of the present invention, the disposable part according to the second aspect of the present invention, the kit according to the third aspect of the present invention and the disposable part and/or the applicator used in the method according to the fourth aspect of the present invention.

The term "patient", as used herein, may relate to a human or an animal, preferably a human. The patient may for example be a pediatric patient, a juvenile patient, or an adult patient, in particular a geriatric patient. The terms "patient" and "subject" maybe used interchangeably. The term "user", as used herein, may refer to a person using or operating the applicator, the disposable part and/or the kit, wherein the user may for example be the patient himself/herself or may, e.g., be a medical practitioner such as a physician or a nurse.

The terms "oral mucosa" and "nasal mucosa", as used herein, may refer to the mucous membrane lining the inside of the oral cavity (mouth) and of the nasal cavity, respectively. Preferably, the mucoadhesive surface is to be arranged on the buccal mucosa, i.e., the mucous membrane lining the inside of the cheeks. In other examples, the mucoadhesive surface is, e.g., to be arranged on the lining mucosa of the oral mucosa, for example on the labial mucosa (i.e., the mucous membrane lining the inside of the lips) or the alveolar mucosa (i.e., the mucous membrane between the labial and buccal mucosae).

The term "mucoadhesive surface", as used herein, may refer to any surface or surface layer that adheres to a mucosa and/or that is configured to adhere to a mucosa, in particular the oral and/or nasal mucosa. In a preferred embodiment, the mucoadhesive surface is provided in the form of a mucoadhesive film, particularly in the form of a mucoadhesive film comprising said mucoadhesive surface (e.g., as a surface layer) and optionally further layer(s). Preferably, the mucoadhesive surface (or film) comprises an active agent (active ingredient) such as a drug (e.g., an active pharmaceutical ingredient), for example a macrolide. The mucoadhesive surface (or film) may be swellable in an aqueous liquid but may be insoluble in the aqueous liquid and/or may have negligible solubility in the aqueous liquid. The insolubility or low solubility may increase the adhesion time on the mucosa and may thus enable that the active agent is released over a long period of time, e.g. for at least 10 min, in some examples at least 30 min, in one example at least 60 min. The mucoadhesive surface (or film) may comprise a mucoadhesive polymer (e.g., crosslinked poly(acrylic acid) (for example polycarbophil or Carbopol), poly[(maleic anhydride)-co-(vinyl methyl ether)] or a salt thereof (for example Gantrez), and/or alginate) and/or a cellulose derivative. Optionally, said mucoadhesive polymer and/or said cellulose derivative, if present, is/are a polymer soluble in an organic solvent. In some examples, the mucoadhesive surface may be a flat surface and may, e.g., not comprise any microneedles or similar structures.

The disposable part may be a single-use item (i.e., may be intended for single use, e.g., a single application of the mucoadhesive film) whereas the applicator preferably is configured to be used multiple times (e.g., with different disposable parts). The applicator may for example be configured for at least 10 applications, preferably at least 100 applications, in some examples at least 1000 applications and in some examples at least 2000 applications, wherein each application may for example comprise arranging the mucoadhesive surface of a respective disposable part on the nasal or oral mucosa. Thereby, the environmental footprint may be minimized. In other examples, the disposable part may be configured to be used multiple times (e.g., with different mucoadhesive surfaces or films) or the applicator may also be a single-use item.

Both the applicator and the disposable part may be specifically adapted for use for medical purposes, e.g., as therapeutic and/or diagnostic products or tools. Both the applicator and the disposable part may be adapted to be safe for oral or nasal application, e.g., comprise or consist of biocompatible materials and/or be free of unsafe and/or potentially harmful (e.g., poisonous or carcinogenic) materials. Both the applicator and the disposable part may be embodied so as to comply with medical device regulations and/or requirements for approval and/or certification of medical devices.

The applicator is a device, in particular a medical device, for arranging a mucoadhesive surface on the nasal or oral mucosa of a patient. The applicator comprises an insertable part and an exterior part connected to the insertable part. The insertable part is configured to be inserted into the nasal or oral cavity of the patient at least in part or in its entirety, e.g., up to a predefined insertion depth. The exterior part is configured to be placed on the skin of the patient (e.g., on outside of the cheek) when the insertable part is inserted into the nasal or oral cavity such that the nasal or oral mucosa is arranged between the insertable part and the exterior part, for example such that the insertable and exterior parts form a clamp or clip around the tissue arranged therebetween. The insertable and exterior parts may face each other with a space therebetween in which the oral or nasal mucosa (along with adjacent tissue bordering or surrounding the nasal or oral cavity, e.g., the cheek and/or the corner of the mouth) is to be arranged. The insertable and exterior parts may for example form a pair of opposing legs or arms. The applicator may comprise a connecting part that mechanically connects the insertable and exterior parts, for example to hold the insertable and exterior parts together (i.e., prevent the insertable and exterior parts from separating or falling apart), e.g., such that the applicator forms a single unit or assembly. In some examples, the applicator may have a substantially U-shaped or H-shaped cross-section with the insertable and exterior parts forming the legs or sides of said "U" and "H", respectively.

The disposable part may comprise a carrier having the mucoadhesive surface. The carrier may provide support, in particular mechanical support, for the mucoadhesive surface. The carrier may for example comprise a substrate (e.g., a slab of material) on which the mucoadhesive surface is arranged. The carrier may for example comprise or consist of a polymer material such as plastic (e.g., a thermoplastic polymer), metal or a combination thereof. The mucoadhesive surface may be arranged on the carrier so as to be exposed at least in part (e.g., not covered by the carrier or other parts of the disposable part). Preferably, the carrier is configured to allow for handling the disposable part without having to touch the mucoadhesive surface. The carrier may for example form a frame or rim around the mucoadhesive surface, e.g., such that the mucoadhesive surface is spaced apart from an outer circumference of the carrier. Providing a carrier may facilitate the handling of mucoadhesive surface or film, in particular for small mucoadhesive surfaces or films, and reduce the risk of damaging the mucoadhesive surface or film. Providing a carrier may furthermore prevent patients from placing the mucoadhesive surface or film on the mucosa in a wrong orientation, e.g., with an active-agent-free backside or backing layer facing the mucosa, thereby avoiding immunosuppression and improving patient safety. By allowing users to handle the disposable part without touching the mucoadhesive surface or film, hygiene may be improved and accidental sticking of the mucoadhesive surface to a user's hand or finger may be avoided.

The disposable part is attached or attachable to the insertable part of the applicator. Preferably, said attachment is temporary, i.e., the disposable part may be removably attached or removably attachable to the insertable part. The terms "removably attached" or "removably attachable", as used herein, may refer to forms of attachment that are intended to be released (e.g., as part of a normal use) without destroying the disposable part and/or the applicator, preferably without requiring the use of any tools (e.g., such that a patient can remove the disposable part by hand). In other examples, said attachment may also be permanent, i.e., the disposable part may be permanently attached or attachable to the insertable part. The terms "permanently attached" or "permanently attachable", as used herein, may refer to forms of attachment that are not intended to be released as part of a normal use but may, e.g., require destroying the disposable part and/or the applicator, and/or the use of special tools for releasing the attachment.

The disposable part is attached or attachable to the insertable part of the applicator such that the mucoadhesive surface of the disposable part faces the nasal or oral mucosa when the nasal or oral mucosa is arranged between the insertable part and the exterior part of the applicator. Put differently, the mucoadhesive surface of the disposable part may face the exterior part of the applicator (e.g., a clamping portion thereof as described below) when the disposable part is attached to the insertable part, e.g., such that the mucoadhesive surface can come in contact with the nasal or oral mucosa when the insertable part is inserted into the nasal or oral cavity of the patient with the exterior part placed on the skin.

For attaching the disposable part to the insertable part of the applicator as described above, one or both of the insertable part of the applicator and the disposable part may be equipped with one or more means configured to attach or retain the disposable part to the insertable part, e.g., to secure, fasten, fix, mount, connect or hold the disposable part to the insertable part. For example, the insertable part may comprise one or more retaining means that are configured to receive the disposable part. Additionally or alternatively, the disposable part may comprise one or more attachment means for attaching the disposable part to the insertable part of the applicator.

The term "retaining means", as used herein, may refer to any means on the applicator side (e.g., being part of or comprised in the applicator) that are configured to attach or retain the disposable part to the insertable part, either own their own or in conjunction with one or more other retaining means on the applicator side and/or one or more attachment means on the disposable part side. The term "attachment means", as used herein, may refer to any means on the disposable part side (e.g., being part of or comprised in the disposable part) that are configured to attach or retain the disposable part to the insertable part, either own their own or in conjunction with one or more retaining means on the applicator side and/or one or more other attachment means on the disposable part side.

The retaining means, if present, and/or the attachment means, if present, may for example be configured to attach or retain the disposable part to the insertable part mechanically (e.g., via interlocking and/or mating components, in particular via a snap fit or a press fit), adhesively/by adhesion (e.g., using an adhesive and/or by creating a negative pressure or vacuum), and/or magnetically (e.g., via interacting magnets). As used herein, a snap fit may for example involve the use of one or more flexible interlocking components such as clips or hooks that may snap in place to achieve attachment. A press fit (also referred to as pressed fit, interference fit or friction fit) may for example involve the use of two or more tightfitting mating parts (e.g., a pin and a corresponding recess) configured to hold together by friction after being pushed together.

The retaining means of the applicator may for example be or comprise a retaining structure that is configured to removably receive the disposable part, in particular an attachment means thereof. The retaining structure may be configured to mechanically retain (e.g., hold or secure) the disposable part, for example via interlocking and/or mating components, in particular via a snap fit or a press fit. The retaining structure may for example be configured to engage (e.g., interlock or mate with) a corresponding counterpart (e.g., an attachment means such as a mechanical connector) on the disposable part to hold the disposable part in place. The retaining structure may, e.g., comprise one or more fasteners such as clips, hooks, clamps or a combination thereof. Additionally or alternatively, the retaining structure may also comprise a mechanical connector such as a pin that is to be received in a corresponding counterpart (e.g., a recess) on the disposable part and/or a recess, a cutout or a hole for receiving a corresponding counterpart (e.g., a mechanical connector such as a pin) on the disposable part, wherein said mechanical connector, recess, cutout or hole may, optionally, be threaded (i.e., comprise a (screwing) thread). Additionally or alternatively, the retaining means may for example be or comprise one or more magnets configured to magnetically hold the disposable part in place, an adhesive for adhesively attaching the disposable part to the applicator, and/or one or more suction cups for holding the disposable part in place. In some examples, the retaining means of the applicator may be embodied as described below for the attachment means of the disposable part.

The attachment means of the disposable part may be configured to be received by the retaining means (e.g., a retaining structure) of the insertable part of the applicator, e.g., to be mechanically, adhesively and/or magnetically retained by the retaining means. In some examples, the attachment means may be or comprise a mechanical connector that is configured to be removably received (e.g., mechanically retained) by the retaining means, for example by a retaining structure as described above. The mechanical connector may in particular be configured to be removably received by the retaining means via a snap fit or a press fit. The mechanical connector may for example be or comprise a pin that is configured to be removably received by the retaining means, e.g., via a snap fit or a press fit. In other examples, the mechanical connector may for example be a threaded mechanical connector (e.g., a threaded pin) and may, e.g., be configured to be screwed into the retaining means (e.g., a threaded recess or hole thereof). Additionally or alternatively, the attachment means may be or comprise an adhesive, e.g., an adhesive layer and/or one or more spots of adhesive. Said adhesive may for example be configured to bind the disposable part to a surface on the insertable part of the applicator. In some examples, the attachment means may for example be or comprise a ring and/or a pair of opposing clips or clamps, which may, e.g., be configured to receive the insertable part (e.g., the clamping portion thereof) therein and therebetween, respectively (for example by inserting the insertable part into the ring and/or between the pair of opposing clips or clamps and/or by snapping or clipping the ring and/or the pair of opposing clips or clamps around the insertable part). In some examples, the attachment means of the disposable part may be configured to attach the disposable part to a finger of a user (e.g., via said ring and/or pair of opposing clips or clamps) in addition to (or instead of) being configured to attach the disposable part to the insertable part of the applicator. This may allow for also using the disposable part without an applicator (i.e., the finger of the user may serve as the "applicator" or "insertable part" thereof).

The mucoadhesive surface may be arranged on a first side of the carrier (e.g., on a top face or surface of the carrier that is to face the exterior part (e.g., the clamping portion thereof) when the disposable part is attached to or received by the applicator). The attachment means may be arranged on a second side of the carrier different from the first side, in particular on a second side of the carrier opposite to the first side (e.g., on a bottom face or surface of the carrier that is to face the insertable part (e.g., the clamping portion thereof) when the disposable part is attached to or received by the applicator). This may facilitate attaching the disposable part to the applicator without the mucoadhesive surface coming in contact with the applicator. In other examples, the attachment means may additionally or alternatively be arranged on a side face of the carrier.

The applicator may comprise means for pressing the mucoadhesive surface of the disposable part against the nasal or oral mucosa (e.g., towards the exterior part of the applicator, in particular a clamping portion thereof) when the disposable part is attached to the insertable part of the applicator (e.g., received by the retaining means of the insertable part and/or attached using the attachment means of the disposable part) and the nasal or oral mucosa is arranged between the insertable part and the exterior part of the applicator. Preferably, the means for pressing the mucoadhesive surface against the nasal or oral mucosa (which may also be referred to as "pressing means" in the following) is or comprises an elastic member, e.g., as detailed below. Additionally or alternatively, the pressing means may for example comprise one or more extendable members (e.g., an inflatable body and/or an extendable piston) and/or one or more magnets, for example a first magnet in the insertable part and a second magnet in the exterior part. In some examples, the pressing means may be or comprise an actuator or motor such as for example an electric actuator or motor but for cost and sustainability reasons, non-actuated pressing means (i.e., pressing means that do not comprise an actuator or motor for actively pressing the mucoadhesive surface against the mucosa but that are, e.g., configured to passively press the mucoadhesive surface against the mucosa without an external source of energy) are preferred, in particular purely mechanical pressing means. Preferably, the pressing means are configured to press the mucoadhesive surface of the disposable part against the nasal or oral mucosa (e.g., are configured to generate a pressing force intrinsically without an external force being applied to the applicator), for example by means of said elastic member, said extendable member and/or said one or more magnets. The pressing means may in particular be configured to press the mucoadhesive surface against the nasal or oral mucosa with a particular force and/or apply a particular pressure between the mucoadhesive surface and the nasal or oral mucosa, e.g., as detailed below. In some examples, the pressing means are additionally or alternatively configured to aid in pressing the mucoadhesive surface against the nasal or oral mucosa, e.g., to relay and/or amplify an external force applied to the applicator, for example by means of a lever. In one example, the external force may be applied by a user, e.g., by pushing the insertable and exterior parts against each other. In such examples, said pressing means may for example be or comprise a hinge and/or an elastic member allowing for the insertable and exterior parts to be moved towards each other, e.g., somewhat similar to a tweezer or forceps.

In a preferred embodiment, the means for pressing the mucoadhesive surface against the nasal or oral mucosa is or comprises an elastic member. The elastic member may be configured to bias (e.g., press, push and/or displace) the disposable part, when received by the retaining means, or a part thereof (e.g., the mucoadhesive surface or film or a frame carrying the mucoadhesive surface or film) towards the exterior part. The elastic member may for example be configured to biased the portion of the insertable part in which the retaining means is arranged (e.g., the clamping portion as described below) towards the exterior part (e.g., the clamping portion thereof). When inserting the insertable part into the nasal or oral cavity, the elastic member may be compressed (e.g., by the user or patient), for example to increase a separation distance between the disposable part, in particular the mucoadhesive surface, and the exterior part (which may also be referred herein to as "opening" the applicator). Once the insertable part has been inserted into the nasal or oral cavity (e.g., up to a predefined insertion distance), the elastic member may be released, e.g., such that the elastic member extends again (for example to its equilibrium configuration or state, i.e., the configuration in the absence of any external forces being applied to the applicator), whereby the separation distance between the disposable part, in particular the mucoadhesive surface, and the exterior part is reduced again (which may also be referred herein to as "closing" the applicator). Thereby, the mucoadhesive surface may be brought in contact with the nasal or oral mucosa (unless such contact was already established earlier, e.g., when inserting the insertable part) and/or may be pressed the mucoadhesive surface against the nasal or oral mucosa. The elastic member may for example be embodied as a spring, for example a coil spring, a cantilever spring and/or a leaf spring.

The insertable part and/or the exterior part of the applicator may comprise(s) a different material than the elastic member. The elastic member may for example comprise a material that is more elastic (e.g., less rigid or stiff) and/or more deformable (e.g., having a higher elastic limit or yield point at which plastic deformation sets in), e.g., for the elastic member to have a higher elasticity than one or both of the insertable and exterior parts (e.g., a smaller spring constant or a smaller Young's modulus). Additionally or alternatively, the insertable part and/or the exterior part of the applicator may also comprise(s) the same material as the elastic member. In addition to or instead of the use of different materials, the elastic member may also be provided with a different elasticity than one or both of the interior and exterior parts by choosing its shape (e.g., a material thickness) appropriately. For example, the material thickness of the elastic member may be lower than the material thickness in one or both of interior and exterior parts. The elastic member, the insertable part, the exterior part and/or the entire applicator may for example comprise or consist of metal, a polymer material such as plastic (e.g., a thermoplastic polymer) or a combination thereof. In one example, the elastic member comprises or consists of a first polymer material and/or a metal and the insertable and exterior parts comprise or consist of a second polymer material different from the first polymer material (e.g., a more rigid polymer material).

The elastic member and one or both of the insertable part and the exterior part, in some examples the applicator in its entirety, are formed as a single piece (e.g., formed integrally). The elastic member and said one or both of the insertable and exterior parts (or the applicator in its entirety) may for example be formed by molding, in particular injection molding. In some examples, the elastic member and said one or both of the insertable and exterior parts (or the applicator in its entirety) may be formed by overmolding and/or multi-material injection molding, in particular two-component injection molding, e.g., to form the insertable part and/or the exterior part of a different material than the elastic member. Other techniques that may additionally or alternatively be used include 3D printing/additive manufacturing techniques, thermoforming and milling, although injection molding is preferred in particular when manufacturing large quantities. In some examples, the applicator may be a multi-piece assembly, wherein some or all of the elastic member, the insertable part and the exterior part may be provided as separate pieces and may be assembled together to form the applicator. In such examples, the same techniques as mentioned above, in particular injection molding, may be used for forming some or all of the elastic member, the insertable part and the exterior part.

The means for pressing the mucoadhesive surface against the nasal or oral mucosa (pressing means) may be configured to press the mucoadhesive surface against the nasal or oral mucosa with a force of between 0.1 N and 50 N, in some examples between 0.5 N and 10 N, in some examples between 2 N and 4 N, preferably between 2.5 N and 3.5 N, most preferably between 2.7 N and 3.3 N, in one example 3.0 N. Additionally or alternatively, the pressing means may be configured to apply a pressure of between 0.1 kPa and 1000 kPa, in some examples between 0.5 kPa and 250 kPa, in some examples between 2.5 kPa and 100 kPa, in some examples between 3 kPa and 60 kPa, most preferably between 4 kPa and 50 kPa, in some examples between 5 kPa and 30 kPa, in some examples between 10 kPa and 20 kPa, preferably between 12.5 kPa and 17.5 kPa, most preferably between 13.5 kPa and 16.5 kPa, in one example 15 kPa between the mucoadhesive surface of said removable disposable part and the nasal or oral mucosa. The aforementioned force and pressure generated by the pressing means may for example refer to the force and pressure, respectively, that is generated by the pressing means at separation distances between the mucoadhesive surface and the exterior part as they typically occur when the nasal or oral mucosa is arranged between the insertable part and the exterior part with the mucoadhesive surface being in contact with the nasal or oral mucosa and the exterior part being placed on the skin of the patient, e.g., at a separation distance of between 2 mm and 25 mm, in some examples between 3 mm and 20 mm, in one example between 5 mm and 15 mm. The present inventors have found that such forces and pressure provide secure adhesion of the mucoadhesive surface to the nasal or oral mucosa and reliable wetting of the mucoadhesive surface but without being perceived as unpleasant or even painful by patients and without risking damage to the nasal or oral mucosa and the mucoadhesive surface.

In some examples, the applicator may comprise a hinge that pivotably connects the insertable part and the exterior part, i.e., such that the insertable and exterior parts may be pivoted or rotated with respect to each other, for example to increase or decrease a separation distance between the mucoadhesive surface and the exterior part. The hinge is not particularly limited and may be any suitable type of hinge or joint known in the art. The hinge may comprise a fulcrum connecting the insertable part and the exterior part so as to allow for a pivoting or rotating motion of the insertable and exterior parts with respect to each other. The hinge may for example comprise an axis or a pin on one of the insertable and exterior parts that is rotatably receive by a corresponding counterpart (e.g., a recess or hole) on the other one of the insertable and exterior parts.

One or both of the insertable part and the exterior part may each comprise a (e.g., proximal or central) hinge portion and a (e.g., distal) clamping portion. Optionally, one or both of the insertable part and the exterior part may also comprise a (e.g., proximal) handle portion. In some examples, the hinge portion may also serve as the handle portion. The clamping portions of the insertable and exterior parts may be biased and/or pressed towards or against each other by the means for pressing the mucoadhesive surface against the nasal or oral mucosa, e.g., by the elastic member.

As used herein, relative terms such as proximal, central and distal may relate to the location of the respective elements as, e.g., seen from a hand of a user operating the applicator, i.e., a proximal portion of the applicator may be closer (or intended to be closer) to the user's hand than a distal portion and a central portion may be arranged between a proximal portion and a distal portion. The insertable part of the applicator may be intended to be inserted with into the nasal or oral cavity with its distal tip or portion first, i.e., with the distal tip or portion facing the nasal or oral cavity.

The retaining means (and thus the disposable part, when attached) may be arranged on and/or in the clamping portion of the insertable part. The handle portion may serve as a handle or grip for a user to operate the applicator. The hinge may connect the hinge portions of the insertable and exterior parts, for example so as to allow for moving the clamping portions apart from each other or vice-versa by pushing the handle portions towards each other and apart from each other, respectively. Thereby, the user may adjust a separation distance between the clamping portions (e.g., between the mucoadhesive surface of the disposable part and the clamping portion of the exterior part) by moving the clamping portions away from each other (to increase the separation distance, i.e., to open the applicator) or towards each other (to decrease the separation distance, i.e., to close the applicator), for example to insert the insertable part into the nasal or oral cavity and/or to arrange the mucoadhesive surface on the nasal or oral mucosa and/or press the the mucoadhesive surface against the nasal or oral mucosa.

The elastic member may be configured to bias the disposable part, when attached, and/or the clamping portion of the insertable part towards the clamping portion of the exterior part, for example such that the disposable part (e.g., the mucoadhesive surface thereof) and/or the clamping portion of the insertable part is in contact with or at a predefined separation distance from the clamping portion of the exterior part when no external force is applied to the handle portions (e.g., when the applicator is not touched by a user and/or the elastic member is released or in its equilibrium state). The predefined separation distance may for example be smaller than a typical thickness of the tissue of the nasal or oral cavity that is to be arranged between the insertable and exterior parts (e.g., a typical thickness of the cheek). The predefined separation distance may for example be between o mm and 10 mm, in some examples between 0.5 mm and 5 mm, in one example between 1 mm and 3 mm.

The elastic member may for example extend between the handle portions of the insertable and exterior parts, wherein the elastic member may, e.g., be configured to push and/or hold the handle portions apart from each other to bias the disposable part, when attached, and/or the clamping portion of the insertable part towards the clamping portion of the exterior part.

In some examples, the hinge may be formed by or comprise the elastic member. The hinge may for example comprise a leaf spring connecting the interior and exterior parts, wherein the leaf spring may serve as the elastic member while at the same time allowing the interior and exterior parts to pivot with respect to each other. In other examples, the hinge may, e.g., comprise a coiled fulcrum as for example used in clothespins, e.g., a coil spring that serves both as the elastic member and as a fulcrum at which the insertable and exterior part can pivot with respect to each other.

The shape of one or both of the applicator and the disposable part may be specifically adapted to the anatomy of the patient, for example the human anatomy, in particular the portion of the nasal or oral mucosa that the mucoadhesive surface is to be arranged on, e.g., the buccal mucosa. This may facilitate arranging the mucoadhesive surface at a defined position on the nasal or oral mucosa, for example on the buccal mucosa or a specific part thereof. For example, physical dimensions of the applicator and/or the disposable part may be chosen so as to facilitate arranging the mucoadhesive surface at such a defined position, for example to prevent arranging the mucoadhesive surface at other positions on the nasal or oral mucosa and/or to guide the mucoadhesive surface towards the defined position. Additionally or alternatively, one or both of the applicator and the disposable part may comprise structural features that are configured to facilitate arranging the mucoadhesive surface at the defined position, for example a guiding structure configured to prevent arranging the mucoadhesive surface at other positions on the nasal or oral mucosa and/or to guide the mucoadhesive surface towards the defined position.

In some examples, the applicator may comprise a contact surface that is arranged between the insertable and exterior parts, wherein the contact surface may be to come in contact with an edge of the nasal or oral orifice of the patient, for example a corner of the mouth, when the insertable part is inserted into the nasal or oral cavity to a predefined insertion depth. By coming in contact with the edge of the nasal or oral orifice, the contact surface may prevent further insertion of the insertable part beyond the predefined insertion depth. Such a contact surface may be an example of the aforementioned guiding structure.

A distance between the contact surface and the retaining means, for example a distance between the contact surface and the mucoadhesive surface (e.g., a center or an edge thereof) of the disposable part when received by the retaining means, may be chosen appropriately to facilitate arranging the mucoadhesive surface at a defined position on the nasal or oral mucosa. The distance between the contact surface and the retaining means may for example be between 0.5 cm and 6 cm, preferably between 2 cm and 4 cm, most preferably between 2.5 cm and 3.5 cm. The present inventors have found that such distances may for example facilitate arranging the mucoadhesive surface on the buccal mucosa for most patients.

In some embodiments, the insertable part comprises a disk-shaped portion in and/or on which the retaining means is arranged. The disk-shaped portion may be or may be comprised in the clamping portion of the insertable part. The disk-shaped portion may for example have a circular or elliptical shape or a polygonal shape with rounded corners (e.g., a square or rectangle with rounded corners, a hexagon with rounded corners or an octagon with rounded corners). A shape, in particular one or more physical dimensions of the disk-shaped portion may be adapted to the anatomy of the patient, for example the human anatomy, in particular the portion of the nasal or oral mucosa that the mucoadhesive surface is to be arranged on, e.g., the buccal mucosa. For example, a lateral physical dimension, in particular a diameter, of the disk-shaped portion may be between 1.5 cm and 5 cm, most preferably between 2.5 cm and 4 cm, in one example between 3.0 cm and 3.5 cm. A lateral physical dimension of the applicator or a part thereof, as used herein, may for example be measured parallel to the mucoadhesive surface (when the disposable part is attached to the applicator) and/or perpendicular to an insertion direction for inserting the insertable part into the oral cavity, e.g., a direction extending from the handle portion to the clamping portion of the insertable part. In some examples, the disposable part, in particular the carrier thereof, may also comprise a disk-shaped portion (e.g., a disk-shaped carrier, in particular a circular carrier) as described above, either in addition to or instead of the disk-shaped portion of the insertable part of the applicator.

The mucoadhesive surface may be formed by a mucoadhesive film (i.e., may for example be a surface of said mucoadhesive film). The mucoadhesive film may be arranged on and/or in the carrier, e.g., attached to a surface of the carrier. The mucoadhesive film may comprise one or more of a mucoadhesive polymer, a cellulose derivative, a plasticizer and/or a colorant, e.g., as described below. Optionally, said mucoadhesive polymer and/or said cellulose derivative, if present, is/are a polymer soluble in an organic solvent. The mucoadhesive film may be a solid preparation comprising a single or multiple layers of suitable material(s) intended to be applied to a body cavity, preferably the buccal cavity. In one embodiment, the mucoadhesive film is an oromucosal patch.

The mucoadhesive film may comprise one or more layers, i.e., may be a single-layer or multi-layer film. The mucoadhesive film may for example comprise one or more of a mucoadhesive polymeric matrix layer, a backing layer (which, e.g., the mucoadhesive polymeric matrix layer may be attached to), an intermediate layer (which may, e.g., be arranged between the mucoadhesive polymeric matrix layer and the backing layer) and an adhesive layer (e.g., for attaching the mucoadhesive film to the carrier), e.g., as detailed below. Each layer of the mucoadhesive film (in particular the mucoadhesive polymeric matrix layer, the backing layer and the intermediate layer) may comprise one or more of a mucoadhesive polymer, a cellulose derivative, a plasticizer and/or a colorant, e.g., as detailed below. The mucoadhesive film may be loaded with an active agent, which may for example be contained in the mucoadhesive polymeric matrix layer.

The mucoadhesive surface (e.g., the mucoadhesive film) may be removably attached to the disposable part (e.g., to the carrier), in particular such that the mucoadhesive surface or film can be separated from the disposable part when the mucoadhesive surface is arranged on the nasal or oral mucosa. The mucoadhesive surface or film may for example be attached to the disposable part by an adhesive, in particular an adhesive layer, wherein the adhesive may, e.g., be provided on the carrier and/or as part of the mucoadhesive film. Preferably, the adhesive is a biocompatible adhesive. In some examples, the adhesive may be water-soluble. The adhesive may for example comprise a water-soluble polymer (e.g., shellac, a vinylpyrrolidone/vinyl acetate copolymer, a polyvinylcaprolactam/polyvinyl acetate/polyethylene glycol copolymer, hydroxypropyl cellulose or hydroxypropyl methyl cellulose and/or polyvinylpyrrolidone). Additionally or alternatively, the adhesive may for example comprise a plasticizer, wherein said plasticizer may, e.g., be or comprise glycerol, polyethylene glycol, in particular polyethylene glycol 200, and/or tributyl citrate.

The adhesive (e.g., the adhesive layer) may for example be configured such that when the disposable part, after pressing the mucoadhesive film against the nasal or oral mucosa by said means for pressing the mucoadhesive surface of the disposable part against the nasal or oral mucosa, is removed from the nasal or oral mucosa (for example by manually compressing the elastic member to open the applicator), the mucoadhesive surface or film separates from the disposable part at the adhesive layer and remains arranged on the nasal or oral mucosa. The adhesive (e.g., the adhesive layer) may have a pull-off force (e.g., at the interface to the mucoadhesive surface or film and/or at the interface to the disposable part or carrier) that is lower than a pull-off force for separating the mucoadhesive film from the nasal or oral mucosa, in particular lower than a pull-off force for separating the mucoadhesive film from the nasal or oral mucosa after the mucoadhesive film was pressed against the nasal or oral mucosa by said pressing means (e.g., after pressing the mucoadhesive film against the nasal or oral mucosa with a force or pressure within the ranges stated above). The pull-off force of an adhesive or adhesive layer, as used herein, may for example refer to the force at which two elements joined by the adhesive or adhesive layer (such as the mucoadhesive film and the carrier) separate when pulled apart from each other with this force (e.g., in a direction normal to the adhesive layer and/or the interface between said elements). The adhesive or adhesive layer may have a pull-off force that is less than 75%, preferably less than 50%, most preferably less than 25% of the pull-off force for separating the mucoadhesive film from the nasal or oral mucosa after the mucoadhesive film was pressed against the nasal or oral mucosa by said pressing means.

As mentioned above, the mucoadhesive film may comprise one or more layers, i.e., may be a single-layer or multi-layer film. The mucoadhesive film may for example comprise a mucoadhesive polymeric matrix layer (which may also be referred to as the "mucoadhesive layer" herein), wherein the mucoadhesive polymeric matrix layer may for example be or form the mucoadhesive surface. The mucoadhesive polymeric matrix layer may comprise a mucoadhesive polymer, e.g., as described below. The polymeric matrix layer may for example comprise alginate. In one embodiment, the mucoadhesive polymeric matrix layer is an alginate layer. In some examples, the mucoadhesive polymeric matrix layer may contain an active agent, e.g. as described below.

Optionally, the mucoadhesive film may comprise a backing layer that the mucoadhesive polymeric matrix layer, when present, may be attached to. The backing layer, when present, may for example be arranged between the mucoadhesive polymeric matrix layer and the adhesive layer for attaching the mucoadhesive film to the carrier. The backing layer may for example be configured to provide support for the polymeric matrix layer and/or to facilitate handling the mucoadhesive film. Preferably, the backing layer is impermeable to water and/or the active agent contained in the polymeric matrix layer, for example so as to prevent release of the active agent from the polymeric matrix layer through the backing layer. Thereby, unidirectional release of an active agent to the oral or nasal mucosa may for example be achieved. The backing layer may for example comprise a water-insoluble polymer and/or a cellulose derivative, for example as described below, e.g. hydroxyethyl cellulose, preferably a water-insoluble cellulose derivative, more preferably ethyl cellulose. Additionally or alternatively, the backing layer may comprise a plasticizer, for example as described below, preferably selected from glycerol, polyethylene glycol, e.g. low molecular weight polyethylene glycol such as PEG 200 or PEG 400, propylene glycol, sorbitol, triacetin, and tributylcitrate, more preferably glycerol.

In some examples, the mucoadhesive film may optionally comprise an intermediate layer between the mucoadhesive polymeric matrix layer and the backing layer, wherein the intermediate layer may for example be configured to facilitate adhesion of the mucoadhesive polymeric matrix layer to the backing layer. Said intermediate layer may comprise a plasticizer, for example as described below, preferably selected from glycerol, polyethylene glycol, e.g. low molecular weight polyethylene glycol such as PEG 200 or PEG 400, propylene glycol, sorbitol, triacetin, and tributylcitrate, more preferably glycerol; and/or a polymer, preferably a polymer comprising polyvinyl acetate and/or polyvinylpyrrolidone, more preferably a vinylpyrrolidone-vinyl acetate copolymer.

In some embodiments, the mucoadhesive surface (or film) may optionally comprise a surfactant, e.g., polysorbate.

The adhesion characteristic of the mucoadhesive surface and/or film may be sufficient for a desired application or retention time, for which the mucoadhesive surface and/or film may be to remain on the oral of nasal mucosa. The application or retention time may for example be at least 1 min, in some examples at least 5 min, in some examples at least 10 min, in one example at least 30 min, and in one example at least 60 min. The application or retention time may for example be between 10 min and 120 min, preferably between 30 min and 60 min. The mucoadhesive surface and/or film may be configured to adhere to the mucosa for at least said application or retention time. In one embodiment, ethyl cellulose is comprised by a mucoadhesive surface and/or film and allows for an application time of at least 30 min, preferably at least 60 min, due to its low water solubility. In one embodiment, the backing layer comprises ethyl cellulose. In one embodiment, the mucoadhesive surface or film is a non-dissolvable surface or film that must be removed from the mucosa, e.g. buccal mucosa, after drug release. In one embodiment, the mucoadhesive film is applied to a patient for a period of at least 30 min.

The mucoadhesive surface and/or the mucoadhesive film may for example have or cover a surface area of 0.5 cm² to 10 cm², preferably 1 cm² to 8 cm², most preferably 2 cm² to 6 cm². In one embodiment, the area weight of the mucoadhesive film is about 20 g/m² to about 300 g/m², preferably about 50 g/m² to about 260 g/m². In one embodiment, the mucoadhesive film has a film thickness of about 20 µm to about 1000 µm, preferably of about 50 µm to about 500 µm, more preferably of about 50 µm to about 300 µm, e.g. about 150 µm to about 260 µm.

A mucoadhesive polymer, as used herein, may relate to a polymer that facilitates and/or mediates mucoadhesion, i.e. adhesion to a mucosa and/or mucus. The mucoadhesive polymer may for example be configured such that attractive forces between the mucoadhesive polymer and the mucosa and/or mucus are generated when the mucoadhesive polymer comes in contact with (e.g., is arranged on) the mucosa and/or mucus. The mucoadhesive polymer may for example be a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof. Preferably, the amphiphilic polymers are selected from polyvinyl caprolactam-polyvinyl acetate-polyethylene glycol graft copolymers, polyoxyl castor oils and D-α-tocopherol-polyethylenglycol-succinate (TPGS), and/or the poly(methacrylates) are selected from neutral protonizable poly(methacrylates) and cationic poly(methacrylates), preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters.

In one embodiment, the mucoadhesive polymer is selected from poly(acrylic acid), e.g. Carbopol^{®} 934 or Carbopol^{®} 971 NF; crosslinked poly(acrylic acid), e.g. polycarbophil; amylopectin, e.g. Proloc^{™} 15; poly(methacrylic acid), e.g. Eudragit^{®} L100 or Eudragit^{®} S100; poly(methacrylate), e.g. Eudragit^{®} E100, Eudragit^{®} RL, or Eudragit^{®} RS; poly[(maleic anhydride)-co-(vinyl methyl ether)] or a salt thereof, e.g. Gantrez^{™} AN 119, Gantrez^{™} AN 139, Gantrez^{™} AN 149, Gantrez^{™} AN 169, or Gantrez^{™} MS-955; gelatine; polysaccharides, e.g. alginates, chitosan, xanthan gum, hyaluronic acid, pectin, or pullulan; cellulose derivatives, e.g. sodium carboxymethylcellulose (CMC), hydroxypropylmethylcellulose (HPMC), hydroxypropylcellulose (HPC), hydroxyethyl methyl cellulose (HEMC), hydroxyethylcellulose (HEC), or methylcellulose (MC); poly(vinyl pyrrolidone), e.g. Kollidon^{®} 30LP or Kollidon^{®} 90F; poly[(vinylpyrrolidone)-co-(vinyl acetate)], e.g. Kollidon^{®} VA64; a mixture of poly(vinylacetate) and poly(vinyl pyrrolidone), e.g. Kollidon^{®} SR; poly(vinyl alcohol); and poly(vinyl acetate).

The term "amphiphilic polymers", as used herein, relates to any amphiphilic polymer, preferably mucoadhesive amphiphilic polymer, known to a person skilled in the art. Typically, an amphiphilic polymer has a hydrophilic (polar) moiety and a hydrophobic (non-polar) moiety. In one embodiment, amphiphilic polymers are selected from polyvinyl caprolactam-polyvinyl acetate-polyethylene glycol graft copolymers, e.g. Soluplus^{®}, polyoxyl castor oils, and D-α-tocopherol-polyethylenglycol-succinate (TPGS). In one embodiment, a hydrophilic polymer is a polymer that contains polar or charged groups. In one embodiment, these groups are non-ionic, anionic, cationic and/or zwitterionic. In one embodiment, the hydrophilic polymer is soluble in water. For example, the hydrophilic polymer can be selected from starch and starch derivatives, dextran, cellulose and cellulose derivatives, such as carboxymethylcellulose, hydroxypropylcellulose, hydroxyethyl cellulose, hydroxypropyl methyl cellulose, hydroxypropylethyl cellulose, sodium carboxymethyl cellulose, ethyl or propyl cellulose, polyacrylic acid, polyacrylate, polyvinylpyrrolidone, polyethylene glycol/polyvinyl alcohol copolymer, polyvinyl alcohol, polyethylene oxide polymers, polyethylene oxide/polyethylene glycol copolymers, polyacrylamide, polyethylene glycol, gelatin, collagen, alginate, pectin, pullulan, traganth, chitosan, alginic acid, arabinogalactan, galactomannan, agar-agar, agarose, carrageenan, shellac, natural gums and/or copolymers thereof. In one embodiment, poly(methacrylates) are selected from neutral protonizable poly(methacrylates) and cationic poly(methacrylates), preferably are selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters. For example, a poly(methacrylate) may be Eudragit^{®} L100, Eudragit^{®} S100, Eudragit^{®} E100, Eudragit^{®} RL, or Eudragit^{®} RS. The term "crosslinked polyacrylic acid polymers", as used herein, relates to polyacrylic acid polymers and crosslinked polyacrylic acid polymers, preferably crosslinked polyacrylic acid polymers, known to a person skilled in the art, such as a homopolymers, copolymers, and interpolymers, e.g. acrylic acid crosslinked with allyl sucrose or allyl pentaerythritol, acrylic acid and C10-C30 alkyl acrylate crosslinked with allyl pentaerythritol, and carbomer homopolymer or copolymer that contains a block copolymer of polyethylene glycol and a long chain alkyl acid ester. For example, a crosslinked polyacrylic acid polymer may be Carbopol^{®} 934 or Carbopol^{®} 971 NF. The term "copolymers of methyl vinyl ether and maleic anhydride", as used herein, relates to any copolymer of methyl vinyl ether and maleic anhydride/maleic acid known to a person skilled in the art, e.g. copolymers of monoalkyl esters of poly (methyl vinyl ether/maleic acid) with varying ester groups. In one embodiment, copolymers of methyl vinyl ether and maleic anhydride are in the form of an anhydride or in a hydrolysed form, such as a mixed sodium and calcium salt of methyl vinyl ether and maleic anhydride e.g. Gantrez^{™} MS-955 polymer. In one embodiment, the copolymers of methyl vinyl ether and maleic anhydride are selected from poly(methyl vinyl ether-co-maleic anhydride), poly(methyl vinyl ether-co-maleic acid), monoethyl ester of poly(methylvinyl ether/maleic acid), mixture of monoethyl ester of poly(methylvinyl ether/maleic acid) and monobutyl ester of poly(methylvinyl ether/maleic acid), and mixed sodium/calcium salts of poly(methylvinyl ether/maleic anhydride). In one embodiment, a copolymer of methyl vinyl ether and maleic anhydride, e.g. Gantrez^{™}, is a mixed sodium and calcium salt of a copolymer of methyl vinyl ether and (hydrolyzed) maleic anhydride. In one embodiment, the copolymer of methyl vinyl ether and maleic anhydride is a complexing agent. For example, a copolymer of methyl vinyl ether and maleic anhydride may be Gantrez^{™} AN 119, Gantrez^{™} AN 139, Gantrez^{™} AN 149, Gantrez^{™} AN 169, Gantrez^{™} AN-903, Gantrez^{™} S-96, Gantrez^{™} S-97, Gantrez^{™} ES-225, Gantrez^{™} ES-425, or Gantrez^{™} MS-955. The terms "polymers comprising polyvinyl acetate and/or polyvinylpyrrolidone" and "polymers comprising polyvinyl acetate and polyvinylpyrrolidone", as used herein, relate to any polymers comprising polyvinyl acetate and/or polyvinylpyrrolidone known to a person skilled in the art, e.g. poly(vinylpyrrolidone), poly(vinylacetate), poly[(vinylpyrrolidone)-co-(vinyl acetate)], and a mixture of poly(vinylacetate) and poly(vinyl pyrrolidone). In one embodiment, the polymer comprising polyvinyl acetate and/or polyvinylpyrrolidone is selected from poly(vinylpyrrolidone), poly(vinylacetate), poly[(vinylpyrrolidone)-co-(vinyl acetate)], e.g. a copolymer of 6 parts polyvinyl pyrrolidone and 4 parts polyvinyl acetate, and a mixture of poly(vinylacetate) and poly(vinyl pyrrolidone), e.g. a 8:2 mixture of poly(vinylacetate) and poly(vinyl pyrrolidone). In one embodiment, a polymer comprising polyvinyl acetate and/or polyvinylpyrrolidone comprises or consists of poly(vinylpyrrolidone). In one embodiment, a polymer comprising polyvinyl acetate and/or polyvinylpyrrolidone comprises or consists of a vinylpyrrolidone-vinyl acetate copolymer, particularly poly[(vinylpyrrolidone)-co-(vinyl acetate)]. In one embodiment, a polymer comprising polyvinyl acetate and/or polyvinylpyrrolidone comprises or consists of a mixture of poly(vinylacetate) and poly(vinylpyrrolidone). In one embodiment, a polymer comprising polyvinyl acetate and/or polyvinylpyrrolidone comprises one or more, preferably multiple, vinyl acetate monomers and/or one or more, preferably multiple, vinylpyrrolidone monomers. For example, polymers comprising polyvinyl acetate and/or polyvinylpyrrolidone may be Kollidon^{®} 30LP, Kollidon^{®} 90F, Kollidon^{®} VA64, or Kollidon^{®} SR.

A cellulose derivative, as used herein, may relate to cellulose and its derivatives. In one embodiment, the cellulose derivative is preferably a cellulose derivative that swells rather than dissolves upon contact with water and/or saliva. The cellulose derivative may for example be selected from hydroxypropyl cellulose (HPC), carboxymethyl cellulose (CMC), e.g. sodium carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof. In one embodiment, the mucoadhesive surface or film comprises a cellulose derivative which is a mucoadhesive cellulose derivative, e.g. selected from hydroxypropyl cellulose (HPC), carboxymethyl cellulose (CMC), hydroxypropyl methylcellulose (HPMC), hydroxyethyl cellulose (HEC), methyl cellulose (MC), and a combination thereof. In one embodiment, a cellulose derivative soluble in an organic solvent is HPC and/or EC. In one embodiment, CMC is soluble in water but not in an organic solvent.

A plasticizer, as used herein, may relate to any plasticizer known to a person skilled in the art, preferably plasticizers which are suitable for in vivo application. The plasticizer may for example be selected from glycerol, polyethylene glycol, e.g. low molecular weight polyethylene glycol such as PEG 200 or PEG 400, propylene glycol, sorbitol, triacetin, and tributylcitrate; more preferably glycerol. In one embodiment, low molecular weight poly(ethylene glycol) has a molecular weight ≤ 600 daltons, preferably ≤ 450 daltons, e.g. 420 daltons. A plasticizer allows for advantageous mechanical properties of the layer(s).

In one embodiment, the mucoadhesive film comprises
a) a mucoadhesive polymer, preferably an amphiphilic polymer; hydroxypropyl cellulose (HPC); carboxymethyl cellulose (CMC); a plasticizer, preferably glycerol; and optionally a colorant, preferably TiO₂;
   preferably 10 wt% - 30 wt% of mucoadhesive polymer; 25 wt% - 35 wt% of hydroxypropyl cellulose (HPC); 10 wt% - 25 wt% of carboxymethyl cellulose (CMC); 10 wt% - 16 wt% of said plasticizer; and optionally 1 wt% - 4 wt% of said colorant; or
b) a mucoadhesive polymer, preferably selected from crosslinked polyacrylic acid polymers and copolymers of methyl vinyl ether and maleic anhydride; hydroxypropyl cellulose (HPC); ethyl cellulose (EC); a plasticizer, preferably glycerol; and optionally a colorant, preferably TiO₂;
   preferably 2 wt% - 17 wt% of mucoadhesive polymer; 30 wt% - 70 wt% of hydroxypropyl cellulose (HPC); 5 wt% - 40 wt% of said ethyl cellulose; 3 wt% - 20 wt% of said plasticizer; and optionally 1 wt% - 5 wt% of said colorant.

In some embodiments, the mucoadhesive film (e.g., at least one of the layers) comprises a colorant, for example TiO2. For example, said mucoadhesive polymeric matrix layer and/or said backing layer may comprise a colorant. In one embodiment, said mucoadhesive polymeric matrix layer and said backing layer each comprise a colorant, wherein said mucoadhesive polymeric matrix layer comprises a first colorant, e.g. a white pigment such as TiO2, and said backing layer comprises a second colorant, e.g. a blue dye such as brilliant blue, wherein said first colorant and said second colorant are different from each other. Advantageously, a colorant in at least one of the layers allows to distinguish between the mucoadhesive polymeric matrix layer and the backing layer. In one embodiment, the mucoadhesive film further comprises a flavoring agent and/or sweetening agent, such as an agent selected from citric acid, peppermint oil, sodium saccharin, and citrus flavor. Advantageously, the flavoring agent and/or sweetening agent enhances the mouthfeel for the patient. Particularly, such flavoring agent and/or sweetening agent efficiently mask(s) an unpleasant taste of the macrolide or other MBF components. In one embodiment, at least one layer further comprises at least one adjuvant selected from the group comprising stabilizing agents, e.g. chelators, colorants, flavorings, sweeteners, taste masking agents, emulsifiers, enhancers, pH regulators, humectants, preservatives and/or antioxidants. In one embodiment, at least one layer comprises a chelator, preferably EDTA. In one embodiment, when referring to a "layer", the mucoadhesive polymeric matrix layer, the intermediate layer, the backing layer and/or the adhesive layer are meant.

Preferably, the mucoadhesive surface or the mucoadhesive film is loaded with an active agent, for example a drug (e.g., an active pharmaceutical ingredient). The active agent may for example be contained in the mucoadhesive polymeric matrix layer of the mucoadhesive film. The mucoadhesive polymeric matrix layer may be configured to release the active agent to the mucosa (e.g., by diffusion), for example when coming in contact with water or saliva. The mucoadhesive surface or film may be intended to be applied to the oral or nasal mucosa to obtain a systemic effect or a local effect, preferably a systemic effect; and/or the mucoadhesive surface or film may be a flexible single-dose preparation configured to be applied to the oral or nasal mucosa to obtain either a systemic or a local effect by delivering the active agent over a certain period of time, after which it is then removed.

The active agent, as used herein, is not particularly limited and may for example be any substance or ingredient providing a biologically active or other effect, in particular other direct effect, on the body of humans or animals, in particular on the structure and/or any function of the body of humans or animals. The active agent may in particular be a drug, for example any substance or ingredient providing a biologically active or other effect in the diagnosis, cure, mitigation, treatment or prevention of a disease or medical condition.

Said active agent may in particular be or comprise a macrolide. The term "macrolide", as used herein, relates to compounds of a class of natural products that comprise or consist of a large macrocyclic lactone ring, e.g. a 14-, 15-, or 16-membered lactone ring, to which one or more deoxy sugars, usually cladinose and desosamine, may be attached. The term "macrolide" may e.g. relate to tacrolimus, sirolimus, everolimus, pimecrolimus, erythromycin, clarithromycin, azithromycin, roxithromycin, josamycin, spiramycin, telithromycin, tylosin, and fidaxomicin, nystatin, natamycin, and amphotericin B. In some embodiments, said macrolide may for example be selected from macrolide immune suppressants, macrolide antibiotics, and macrolide antifungals, wherein, preferably,
- the macrolide immune suppressant is selected from tacrolimus, sirolimus, everolimus, and pimecrolimus;
- the macrolide antibiotic is selected from erythromycin, clarithromycin, azithromycin, roxithromycin, josamycin, spiramycin, telithromycin, tylosin, and fidaxomicin; and/or
- the macrolide antifungal is selected from polyenes, in particular nystatin, natamycin, and amphotericin B.

In a preferred embodiment, the macrolide is selected from tacrolimus, sirolimus, everolimus, and pimecrolimus, preferably is selected from tacrolimus and sirolimus, more preferably is tacrolimus. In some embodiments, said macrolide may be for use in a method of preventing or treating an immunological disease or disorder, preferably a transplant rejection.

In one embodiment, said macrolide is present in said mucoadhesive polymeric matrix layer at a concentration of at least 2 wt%, preferably at least 3 wt%, e.g. 3.2 wt%. Said macrolide may be nanoparticulate macrolide or non-nanoparticulate macrolide, preferably micronized crystalline non-nanoparticulate macrolide and/or molecularly dissolved non-nanoparticulate macrolide. In one embodiment, said micronized macrolide has an average particle size of from 1 µm to 100 µm, preferably of from 1.5 µm to 50 µm, more preferably of from 2 µm to 25 µm. In one embodiment, said non-nanoparticulate macrolide is present in said mucoadhesive polymeric matrix layer in the molecularly dissolved non-nanoparticulate form; and, optionally, said mucoadhesive polymer and/or said cellulose derivative, if present, is/are a polymer soluble in an organic solvent.

In one embodiment, the active agent is a hydrophobic active agent, preferably a hydrophobic drug that is insoluble in water and aqueous solutions and that is, more preferably, selected from macrolides, non-steroidal anti-inflammatory drugs, hormones, antibodies, steroids, anticancer agents, opioid drugs, and mixtures thereof, wherein more preferably said macrolide is selected from macrolide immune suppressants, macrolide antibiotics, and macrolide antifungals; wherein more preferably, said hydrophobic active pharmaceutical ingredient (API) is selected from tacrolimus, sirolimus, everolimus, pimecrolimus, erythromycin, clarithromycin, azithromycin, roxithromycin, josamycin, spiramycin, telithromycin, tylosin, fidaxomicin, nystatin, natamycin, amphotericin B; in particular, acetylsalicylic acid, ibuprofen, dexibuprofen, flurbiprofen, naproxen, ketoprofen, tiaprofenic acid, diclofenac, indomethacin, acemetacin, flufenamic acid, mefenamic acid, oxicams, e.g. piroxicam, tenoxicam, meloxicam, lornoxicam, nabumeton, rofecoxib, parecoxib, etoricoxib, celecoxib, alpha-atrial natriuretic peptide, arginine vasopressin, atropine, augrnerosen, atorvastatin, bevacizumab, calcitonins, chorionic gonadotropins, corticotropin, desmopressin, epibatidine, cetuxirnab, exenatide, trastuzumab, adalimumab, human insulin, ketoconazole, lanreotide, lutropin alpha, metoprolol, minoxidil, nesiritide, octreotide, paclitaxel, paracetamol, pegaptanib, recombinant follicle stimulating hormone, recombinant growth factors, infliximab, rituximab, sermorelin, somatotropin, a taxane derivative, taxol, teriparatide acetate, thyrotropin, triclosan, urofollitropin, omalizumab, actinomycin D, albendazole, aldosterone, alprazolam, amiodarone, amitriptyline, amprenavir, asimadoline, atorvastatin, bunitrolol, buspirone, camptothecin, carbamazepine, carvedilol, celiprolol, cyclosporine A, cimetidine, clotrimazole, colchicine, cortisone, daunorubicin, debrisoquine, dexamethasone, diazepam, digitoxin, digoxin, diltiazem, docetaxel, domperidone, doxorubicin, efavirenz, epirubicin, erythromycin, ergotamine, estradiol, estradiol glucuronide, erlotinib, etoposide, phenytoin, fentanyl, felodipine, phenothiazines, fexofenadine, fluoroquinolones, fluorouracil, gentamicin, griseofulvin, hydrocortisone, imatinib, indinavir, itraconazole, ivermectin, ketoconazole, kaempferol, levofloxacin, lidocaine, loperamide, losartan, lovastatin, mebendazole, methylprednisolone, methotrexate, mibefradil, midazolam, nisoldipine, morphine, nelfinavir, nicardipine, nitrendipine, nifedipine, ondansetron, paclitaxel, pentazocine, praziquantel, prednisolone, prednisone, quercetin, quinidine, ranitidine, rifabutin, rifampicin, ritonavir, saquinavir, sulfame-thizole, tamoxifen, talinolol, teniposide, terfenadine, tetracycline, topotecan, triamcinolone, valspodar, verapamil, vinblastine, vincristine, vindesine, zopiclone, and mixtures thereof.

In one embodiment, the hydrophobic active agent is a macrolide, and the macrolide is selected from macrolide immune suppressants, macrolide antibiotics, and macrolide antifungals, wherein the macrolide immune suppressant is selected from tacrolimus, sirolimus, everolimus and pimecrolimus; the macrolide antibiotic is selected from erythromycin, clarithromycin, azithromycin, roxithromycin, josamycin, spiramycin, telithromycin, tylosin, and fidaxomicin; and the macrolide antifungal is selected from polyenes, in particular nystatin, natamycin, and amphotericin B.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.
**Figures 1a, 1b** show schematic illustrations of a kit comprising a disposable part with a mucoadhesive surface that is attached to an applicator for arranging said mucoadhesive surface on the nasal or oral mucosa of a patient according to an example in side view.
**Figure 2a** shows a schematic illustration of a kit comprising a disposable part and an applicator according to another example in side view, wherein the disposable part comprises an adhesive for attaching the disposable part to the applicator. **Figure 2b** shows a close-up view of the disposable part of the kit of Figure 2a.
**Figure 3** shows a schematic illustration of a kit comprising a disposable part and an applicator according to another example in side view, wherein the applicator comprises a retaining structure configured to removably receive a mechanical connector of the disposable part.
**Figures 4a, 4b** show schematic illustrations of a kit comprising a disposable part and a multi-piece applicator according to another example in a perspective view.
**Figures 5a to 5g** show schematic illustrations of a kit comprising a disposable part and a single-piece applicator according to another example.
**Figure 6** shows a flow chart of a method for arranging a mucoadhesive surface on the nasal or oral mucosa of a patient in accordance with an example.

In the following, reference is made to these examples, which are given to illustrate, not to limit the present invention. Corresponding elements may be denoted using the same reference sign throughout the figures without necessarily implying any particular relation between these elements even though these elements may also be identical or similar in at least some embodiments.

### EXAMPLES

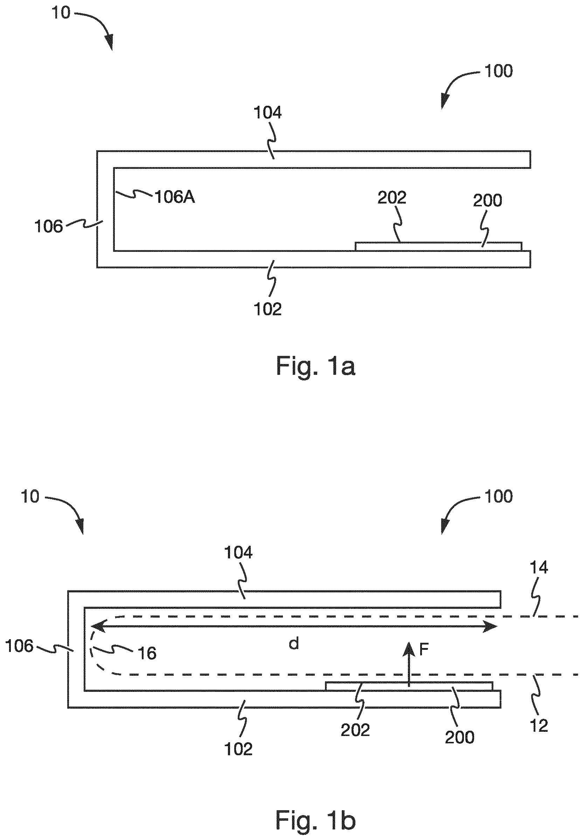

**Figures 1a, 1b** show schematic illustrations of a kit 10 comprising a disposable part 200 with a mucoadhesive surface 202 and an applicator 100 for arranging the mucoadhesive surface 202 on the nasal or oral mucosa 12 of a patient in accordance with an example. The kit 10 as such is depicted in Fig. 1a whereas Fig. 1b depicts the kit 10 with its insertable part 102 inserted into the nasal or oral cavity of the patient and its exterior part 104 placed on the skin 14 of the patient. The kit 10 may for example be used for arranging the mucoadhesive surface 202 on the buccal mucosa, which is used as a non-limiting example for illustration purposes in the following. In other examples, the kit 10 may additionally or alternatively also be used for arranging the mucoadhesive surface 202 on other portions of the nasal or oral mucosa.

The applicator 100 comprises an insertable part 102 and an exterior part 104 that is connected to the insertable part 102 through a connecting part 106, thereby forming a substantially U-shaped clamp with two opposing legs or arms formed by the insertable and exterior parts 102, 104. The insertable part 102, the exterior part 104 and the connecting part 106 define an opening or open space therebetween, which is configured to accommodate the tissue bordering or surrounding the oral cavity, in particular the corner of the mouth and the cheek. The distal portions of the insertable and exterior parts 102, 104 facing away from the connecting part 106 may also be referred to as the clamping portions of the applicator 100. As illustrated in Fig. 1b, the insertable part 102 is configured to be inserted into the oral (or nasal) cavity of the patient, in particular such that the mucoadhesive surface 202 of the disposable part 200 comes in contact with the buccal mucosa 12. The exterior part 104 is configured to be placed on (e.g., in contact with) the skin 14 on the outside of the cheek such that the buccal mucosa 12 is arranged between the insertable part 102 and the exterior part 104.

The insertable part 102, the exterior part 104, the connecting part 106 and/or the entire applicator 100 may, e.g., comprise or consist of a polymer material such as plastic, in particular a thermoplastic polymer such as for example polypropylene (PP), polyethylene (PE, e.g., low-density or high-density polyethylene), polyethylene terephthalate (PET), polyvinyl chloride (PVC), polyamide (PA), acrylonitrile butadiene styrene (ABS), polyhydroxyalkanoates (PHA), polyactic acid (PLA) or a combination thereof. Preferably, said polymer material is a partially or fully bio-based material (e.g., a material obtained from renewable resources such as algae, bacteria, microorganisms and/or plants at least in part or in its entirety), for example bio-based PP, bio-based PE, bio-based PET, bio-based PLA or a combination thereof. Additionally or alternatively, said polymer material may be a partially or fully recycled material, for example recycled PE, recycled PP, recycled ABS or a combination thereof. In some examples, said polymer material may comprise cellulose, for example between 5% and 75%, in some examples between 10% and 50%, in one example between 20% and 40% cellulose. In some examples the insertable part 102, the exterior part 104 the connecting part 106 and/or the entire applicator 100 may also comprise one or more other materials in addition or instead of the polymer material, for example a metal.

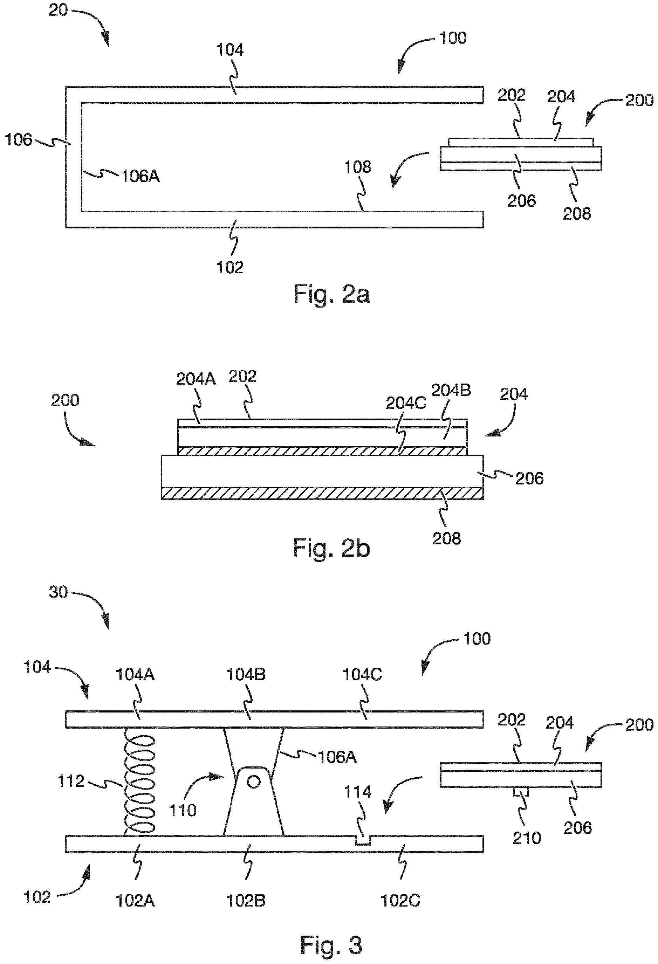

The disposable part 200 comprises a mucoadhesive surface 202, which may for example be formed by a mucoadhesive film, in particular a mucoadhesive film loaded with an active agent, e.g., as described below with reference to Figs. 2a, 2b. In some examples the disposable part 200 may be embodied as the disposable part of any one of the kits 20, 30, 40, 50 described below with reference to Figs. 2 to 5.

In the example of Figs. 1a, 1b, the disposable part 200 is attached to the insertable part 102 of the applicator 100. In other examples, the applicator 100 and the disposable part 200 may be provided as separate units or devices with the disposable part 200 being attachable to the insertable part 102, for example as described below with reference to Figs. 2 to 5. The disposable part 200 is attached to the insertable part 102 such that the mucoadhesive surface 202 of the disposable part 200 faces the exterior part 104, in particular the distal clamping portion thereof, and thus the buccal mucosa 12 when the buccal mucosa 12 is arranged between the insertable part 102 and the exterior part 104 of the applicator 100.

In some examples, the disposable part 200 may be attached removably to the insertable part 102, for example such that the applicator 100 may be used multiple times with a different disposable part 200 for each application. The disposable part 200 may for example be attached to the insertable part 102 using an adhesive, e.g., as detailed below with reference to Figs. 2a, 2b. In some examples, the disposable part 200 may be dissolvable in its entirety. In some examples, the applicator 100 may also be a single-use item, wherein the disposable part 200 may, e.g., be attached permanently to the insertable part 102.

The connecting part 106 forms a contact surface 106A that extends between the insertable and exterior parts 102, 104 and faces the opening between the insertable and exterior parts 102, 104. When the insertable part 102 is inserted into the oral cavity up to a predefined insertion depth d, the contact surface 106A comes in contact with the corner 16 of the mouth as illustrated in Fig. 1b, thereby preventing further insertion of the insertable part 102 beyond the predefined insertion depth d. This may facilitate arranging the mucoadhesive surface 202 on a certain portion of the oral (or nasal) mucosa, for example on the buccal mucosa or a portion thereof. For arranging the mucoadhesive surface 202 on the buccal mucosa, the insertion depth d may for example be between 1 cm and 7 cm, preferably between 2.5 cm and 5 cm, most preferably between 3 cm and 4.5 cm. The predefined insertion depth may for example be defined or set by choosing a shape of the contact surface 106A and/or a length of the insertable part 102 accordingly. In one example, the length of the insertable part (e.g., from the contact surface 106A to a distal tip of the insertable part 102) may correspond to (e.g., be equal or substantially equal to) the predefined insertion depth.

The applicator 100 comprises a means for pressing the mucoadhesive surface 202 of the disposable part 200 against the buccal mucosa 12 when the buccal mucosa 12 is arranged between the insertable and exterior parts 102, 104 of the applicator 100 as illustrated by the arrow labeled F in Fig. 1b. In the example of Figs. 1a, 1b, some or all of the insertable part 102, the exterior part 104 and the connecting part 106 are formed of an elastic material at least in part. The respective parts of the applicator 100 thus form an elastic member, which biases the disposable part 200 (e.g., the distal clamping portion of the insertable part 102 to which the disposable part 200 attached) towards the exterior part 104 of the applicator 100 (e.g., the distal clamping portion thereof) and thereby provides said pressing means.

The applicator 100 may for example be formed such that the insertable and exterior parts 102, 104 (e.g., the distal clamping portions thereof) and/or the disposable part 200 and the exterior part 104 are held at a predefined separation distance (with no external force applied), which may be smaller than a typical thickness of the cheek. The predefined separation distance may for example be between 0 mm and 10 mm, in some examples between 0.5 mm and 5 mm, in one example between 1 mm and 3 mm. For inserting the insertable part 102, a user may manually force the insertable and exterior parts 102, 104 further apart, thereby opening the applicator. Once the insertable part 102 has been inserted into the oral cavity, e.g., up to the predefined insertion depth d, the user may release the insertable and exterior parts 102, 104, whereby the elastic member may push the insertable and exterior parts 102, 104 (e.g., the clamping portions thereof) towards each other, thus pressing the mucoadhesive surface 202 against the buccal mucosa 12. The elastic member may for example be configured to press the mucoadhesive surface 202 against the buccal mucosa 12 with a force of between 2 N and 4 N, preferably between 2.5 N and 3.5 N, most preferably between 2.7 N and 3.3 N, in one example 3.0 N. Additionally or alternatively, the elastic member may be configured to apply a pressure of between 10 kPa and 20 kPa, preferably between 12.5 kPa and 17.5 kPa, most preferably between 13.5 kPa and 16.5 kPa, in one example 15 kPa between the mucoadhesive surface 202 and the buccal mucosa 12.

**Figure 2a, 2b** show schematic illustrations of a kit 20 comprising a disposable part 200 having a mucoadhesive surface 202 and an applicator 100 for arranging the mucoadhesive surface 202 on the nasal or oral mucosa, in particular the buccal mucosa of a patient (not shown), in accordance with another example. Fig. 2a depicts the entire kit 20 whereas Fig. 2b shows a close-up view of the disposable part 200 of the kit 20.

The applicator 100 of the kit 20 is similar to the applicator of the kit 10 in Figs. 1a, 1b and also comprises an insertable part 102, an exterior part 104 and a connecting part 106, which may be embodied as described above for the kit 10.

The disposable part 200 of the kit 20 comprises a mucoadhesive film 204, the upper surface (facing away from the carrier 206) of which forms the mucoadhesive surface 202. In the example of Fig. 2b, the mucoadhesive film 204 comprises a mucoadhesive polymeric matrix layer 204A (forming the mucoadhesive surface 202) and a backing layer 204B that the mucoadhesive polymeric matrix layer 204A is attached to. The mucoadhesive polymeric matrix layer 204A may comprise a mucoadhesive polymer such as crosslinked poly(acrylic acid) (for example polycarbophil or Carbopol), poly[(maleic anhydride)-co-(vinyl methyl ether)] or a salt thereof (for example Gantrez), and/or alginate. In some examples, the mucoadhesive polymeric matrix layer 204A may contain an active agent, in particular a drug such as a macrolide. The backing layer 204B may for example comprise one or more of a water-insoluble polymer, a cellulose derivative and a plasticizer. Preferably, the backing layer 204A is impermeable to the active agent contained in the mucoadhesive polymeric matrix layer 204A. In some examples, one or both of the mucoadhesive polymeric matrix layer 204A and the backing layer 204B may comprise a colorant, for example to allow a user to distinguish the two layers from each other.

The disposable part 200 further comprises a carrier 206, to which the mucoadhesive film 204 is attached through an adhesive is in the form of an adhesive layer 204C. The adhesive layer 204C may for example be provided as part of the mucoadhesive film 204 and/or as part of the carrier 206. The carrier 206 may be configured to support the mucoadhesive film 204 and may for example comprise or consist of a rigid or semi-rigid material. The disposable part 200 and/or the carrier 206 may, e.g., comprise or consist of a polymer material such as plastic, in particular a thermoplastic polymer such as for example polypropylene (PP), polyethylene (PE, e.g., low-density or high-density polyethylene), polyethylene terephthalate (PET), polyvinyl chloride (PVC), polyamide (PA), acrylonitrile butadiene styrene (ABS), polyhydroxyalkanoates (PHA), polyactic acid (PLA) or a combination thereof. Preferably, said polymer material is a partially or fully bio-based material (e.g., a material obtained from renewable resources such as algae, bacteria, microorganisms and/or plants at least in part), for example bio-based PP, bio-based PE, bio-based PET, bio-based polyactic acid (PLA) or a combination thereof. Additionally or alternatively, said polymer material may be a partially or fully recycled material, for example recycled PE, recycled PP, recycled ABS or a combination thereof. In some examples, said polymer material may comprise cellulose, for example between 5% and 75%, in some examples between 10% and 50%, in one example between 20% and 40% cellulose. In some examples, the disposable part 200 and/or the carrier 106 may also comprise one or more other materials in addition or instead of the polymer material, for example a metal.

In contrast to the kit 10 of Figs. 1a, 1b, the applicator 100 and the disposable part 200 of the kit 20 are provided separate from each other, for example for assembly by a user. For this, an attachment means 208 is provided on the disposable part 200 for attaching the disposable part 200 to the insertable part 102 of the applicator 100. In the example of Figs. 2a, 2b, the attachment means 208 is formed by an adhesive 208, which may for example be arranged as a contiguous layer or spatially separated spots on a bottom side or surface of the disposable part 200, e.g., on a bottom side or surface of the carrier 206, opposite a top side or surface of the disposable part 200 and the carrier 206, respectively, on which the mucoadhesive film 204 is arranged.

The insertable part 102 of the applicator 100 comprises an attachment surface 108 for arranging the disposable part 200 on. In some examples, the attachment surface 108 may be specifically adapted to facilitate adhesion of the adhesive 208, e.g., have a suitable degree of surface flatness and/or a suitable surface finish or coating. Such an attachment surface may, e.g., constitute another example of a retaining means. The attachment surface 108 is arranged such that when the disposable part 200 is placed on (e.g., adheres to) the attachment surface 108 with the adhesive 208 therebetween, the mucoadhesive film 204 with the mucoadhesive surface 202 faces the exterior part 104.

The adhesive 208 and the adhesive in the adhesive layer 204C may be configured so as to allow for separating the mucoadhesive film 204 from the disposable part 200 while the carrier 206 remains attached to the applicator 100. In particular, the adhesive in the adhesive layer 204C may have a lower pull-off force than the adhesive 208, e.g., such that the adhesive layer 204C forms an intended breaking point at which the mucoadhesive film 204 may be separated from the carrier 206 and the applicator 100. Preferably, the adhesive in the adhesive layer 204C has a pull-off force that is lower than the pull-off force for separating the mucoadhesive film 204 from the buccal mucosa 12 after the mucoadhesive film 204 was pressed against the buccal mucosa 12 by the elastic member/pressing means of the applicator 100. This may allow for removing the applicator 100 from the oral cavity after arranging the mucoadhesive film 204 on the buccal mucosa 12 with the mucoadhesive film 204 remaining on the buccal mucosa 12. In some examples, the adhesive layer 204C may be configured to loose adhesive strength (e.g., exhibit a lower pull-off force) when coming in contact with water and/or saliva. The adhesive layer 204C may, e.g., be configured to dissolve at least in part or in its entirety when coming in contact with water and/or saliva. Preferably, one or both of the adhesive in the adhesive layer 204C and the adhesive 208 is/are a biocompatible adhesive.

**Figure 3** shows a schematic illustration of a kit 30 comprising a disposable part 200 with a mucoadhesive surface 202 and an applicator 100 for arranging the mucoadhesive surface 202 on the nasal or oral mucosa, in particular the buccal mucosa of a patient (not shown), according to another example.

In this example, the applicator 100 also comprises an insertable part 102 and an exterior part 104, each of which comprises a proximal handle portion 102A, 104A, a central hinge portion 102B, 104B and a distal clamping portion 102C, 104C. The applicator 100 further comprises a hinge 110, which is arranged between the central hinge portions 102B, 104B, e.g., such that the applicator 100 substantially assumes the shape of an "H" as illustrated in Fig. 3. The hinge 110 connects the insertable part 102 and the exterior part 104 and is configured to allow for a pivoting motion of the insertable part 102 and the exterior part 104 with respect to each other. Thereby, the applicator 100 may be opened (i.e., the clamping portions 102C, 104C may be moved apart from each other) by moving the handle portions 102A, 104A towards each other (e.g., pushing the handle portions 102A, 104A together) and closed (i.e., the clamping portions 102C, 104C may be moved towards each other) by moving the handle portions 102A, 104A away from each other (e.g., pushing the handle portions 102A, 104A apart). The hinge 110 may form a contact surface 106A for limiting the insertion depth of the insertable part 102 similar to the contact surface of the applicator of the kit 10 in Figs. 1a, 1b.

The applicator 100 further comprises an elastic member 112 (as an example of a means for pressing the mucoadhesive surface 202 against the buccal mucosa 12), which in this example is embodied as a coil spring and may for example be made of metal and/or plastic. The elastic member 112 is arranged between and anchored to the handle portions 102A, 104A. The elastic member 112 is configured to push or hold the handle portions 102A, 104A apart from each other, thereby biasing the clamping portions 102C, 104C towards each other, e.g., to bring the clamping portions 102C, 104C in contact with each other or to within a predefined separation distance when the elastic member 112 is not compressed by an external force, e.g., by a user. As mentioned above, said predefined separation distance may be smaller than a typical thickness of the cheek for pressing the mucoadhesive surface 202 of the disposable part against the buccal mucosa. In this way, the applicator 100 may be configured to close automatically whenever the elastic member 112 is released (e.g., no longer compressed by an external force), for example whenever a user releases the handle portions 102A, 104A.

The disposable part 200 is embodied similar to the disposable part of the kit 20 of Figs. 2a, 2b and also comprises a mucoadhesive film 204 having a mucoadhesive surface 202 arranged on a carrier 206. In the example of Fig. 3, however, the disposable part 200 can be attached to the applicator 100 by mechanical means instead of (or in addition to) the adhesive 208 of the kit 20. For this, the applicator 100 comprises a retaining means 114 that is configured to receive a corresponding attachment means 210 of the disposable part 200. The retaining means 114 is embodied as a retaining structure that is configured to receive and mechanically retain the disposable part 200. As illustrated in Fig. 3, the retaining structure 114 may for example be or comprise a recess that is configured to receive a corresponding counterpart on the disposable part 200 via a press fit. The attachment means 210 of the disposable part 200 is embodied as a mechanical connector forming said corresponding counterpart. The mechanical connector may for example comprise or be a pin protruding from a bottom surface or side of the disposable part opposite to the mucoadhesive surface 202 as also illustrated in Fig. 3.

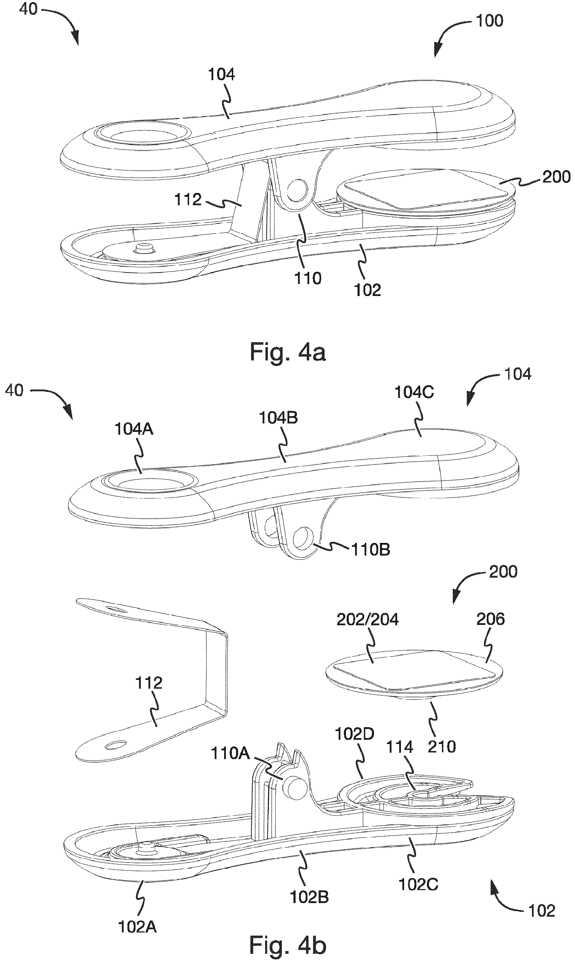

**Figures 4a, 4b** show schematic illustrations of a kit 40 comprising a disposable part 200 with a mucoadhesive surface 202 and an applicator 100 for arranging the mucoadhesive surface 202 on the nasal or oral mucosa, in particular the buccal mucosa of a patient (not shown) according to another example. Fig. 4a shows a perspective view of the kit 40 in an assembled state and Fig. 4b shows an exploded view of the kit 40 in a disassembled state.

The applicator 100 of the kit 40 is similar to the applicator of the kit 30 in Fig. 3 and also comprises an insertable part 102 and an exterior part 104, which are pivotally connected by a hinge 110 with an elastic member 112 biasing clamping portions 102C, 104C of the insertable and exterior parts 102, 104 towards each other. The applicator 110 of the kit 40 is a multi-piece assembly, wherein some or all of the insertable part 102, the exterior part 104, the hinge 110 and the elastic member 112 may be provided as separate units or members.

The hinge 110 comprises an axis 110A arranged on a protruding basis in the handle portion 102B of the insertable part 102, wherein the axis 110A may for example be formed by a pair of pins protruding from opposite side faces of the basis. The hinge 110 further comprises a pair of holes 110B that are configured to rotatably receive the axis 110A and are arranged on a protruding basis in the handle portion 104B of the exterior part 104.

In this example, the elastic member 112 is embodied as a leaf spring that is to be arranged between the handle portions 102A, 104A of the insertable and exterior parts 102, 104. The leaf spring 112 may for example comprise a pair of holes that are to be received by a pair of pins arranged on the handle portions 102A, 104A for securing the leaf spring 112 to the applicator as illustrated in Figs. 4a, 4b. The leaf spring 112 may for example be configured such that the clamping portions 102C, 104C are closer together than the handle portions 102A, 104A when the leaf spring 112 is not compressed by an external force. Put differently, the handle portions 102A, 104A may have to be pushed together to compress the leaf spring 112 for aligning the insertable and exterior parts 102, 104 parallel to each other as illustrated in Fig. 4a. The leaf spring 112 may for example comprise or consist of metal, a polymer material such as plastic or a combination thereof.

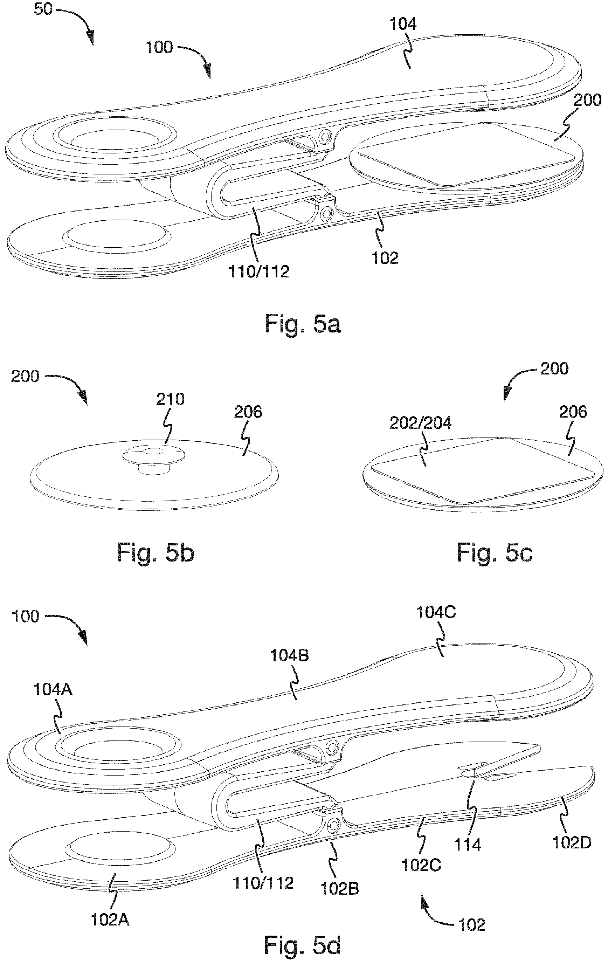

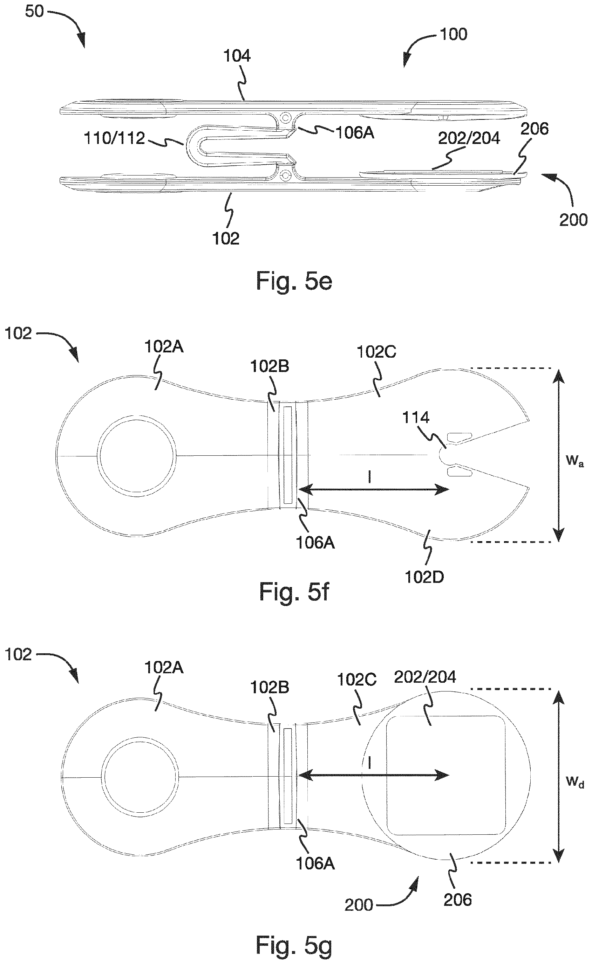

The disposable part 200 of the kit 40 comprises a carrier 206 having a circular top surface on which a mucoadhesive film 204 with the mucoadhesive surface 202 is arranged, e.g., as described above for the kit 20 of Figs. 2a, 2b. A mechanical connector 210 (as an example of an attachment means) in the form of a protruding pin is arranged on a bottom surface of the carrier 206 opposite the top surface, wherein the protruding pin may for example be embodied similar to the one shown in Fig. 5b. A retaining structure 114 (as an example of a retaining means) configured to receive the mechanical connector 210 of the disposable part 200 is arranged in the clamping portion 102C of the insertable part 102, in particular in a disk-shaped portion 102D thereof. The retaining structure 112 is embodied as a tapered cutout with a circular receiving end similar to the retaining structure shown in Fig. 5f.

**Figures 5a to 5g** show schematic illustrations of a kit 50 comprising a disposable part 200 having a mucoadhesive surface 202 and an applicator 100 for arranging the mucoadhesive surface 202 on the nasal or oral mucosa, in particular the buccal mucosa of a patient (not shown) according to another example. Fig. 5a and 5e show a perspective view and a side view, respectively, of the applicator 100 with the disposable part 200 attached thereto. Figs. 5b and 5c show perspective views of a bottom side and a top side, respectively, of the disposable part 200. Fig. 5d shows a perspective view of the applicator 100 without the disposable part 200. Figs. 5f and 5g shows top views of the insertable part 102 with and without, respectively, the disposable part 200 attached thereto.

The kit 50 is similar to the kit 40 of Figs. 4a, 4b except that the applicator 100, which comprises the insertable part 102, a combined elastic member/hinge 110, 112 and the exterior part 104, may be formed integrally as a single piece in this example. The applicator 100 may for example be formed by injection molding, in particular two-component injection molding, wherein the elastic member/hinge 110, 112 may for example be formed of a first material (e.g., a first polymer material such as a first thermoplastic polymer material) and the insertable and exterior parts 102, 104 of a second material (e.g., a second polymer material such as a second thermoplastic polymer material) different from the first material, e.g., a less elastic material. The combined elastic member/hinge 110, 112 may be configured to both bias the clamping portions 102C, 104C of the insertable and exterior parts 102, 104 towards each other and allow for a pivoting motion of the insertable and exterior parts 102, 104 with respect to each other. The combined elastic member/hinge 110, 112 may for example be or comprise a leaf spring. Optionally, the leaf spring may be anchored rotatably in one or two corresponding bearings in the insertable and/or exterior parts 102, 104, in particular in examples where the applicator 100 is not formed integrally as a single piece but provided as a multi-piece assembly, e.g. similar to the kit 40 of Figs. 4a, 4b.

The disposable part 200 of the kit 50 comprises a carrier 206 with a mucoadhesive film 204 having a mucoadhesive surface 202 being arranged on a top surface thereof. The mucoadhesive film 204 may for example have a quadratic or rectangular shape and may be dimensioned such that the carrier 206, which may for example have a circular shape, forms a frame or rim around the mucoadhesive film 204 as illustrated in Fig. 5c, thereby allowing for handling the disposable part 200 without touching the mucoadhesive film 204. A surface area of the mucoadhesive surface 202 may for example be between 0.5 cm² to 10 cm², preferably 1 cm² to 8 cm², most preferably 2 cm² to 6 cm².

A mechanical connector 210 (as an example of an attachment means) is arranged on a bottom surface of the carrier 206. In the example of Fig. 5b, the mechanical connector 210 is a pin protruding from the bottom surface of the carrier 206, wherein the pin may optionally comprise a widened end portion forming a collar or flange around the pin as illustrated in Fig. 5b.

As a corresponding retaining structure (as an example of a retaining means), the insertable part 102 comprises a cutout 114 that is configured to receive the mechanical connector 210. The cutout 114 comprises a tapered portion, e.g., to facilitate insertion of the mechanical connector 210, with a circular end portion (receiving end) arranged at the end thereof. The circular end portion of the cutout 114 may be configured to retain the mechanical connector 210 via a snap fit. The circular end portion may for example have a diameter that is slightly larger than a width of the tapered portion adjacent to the circular end portion, thereby forming a pair of protrusions for retaining the mechanical connector 210 within the circular end portions. Optionally, as illustrated in Fig. 5f, one or more structural weak spots such as holes or recesses may be provided adjacent to said pair of protrusions, e.g., to facilitate insertion of the mechanical connector 210 into the circular end portion by deforming the protrusions and/or moving the protrusions apart.

The combined elastic member/hinge 110, 112 may form a contact surface 106A that is to come in contact with a corner of the mouth when inserting the insertable part 102 into the oral cavity to a predefined insertion depth, thereby preventing insertion of the insertable part 102 beyond said insertion depth, e.g., similar as described above. Preferably, the predefined insertion depth is such that the mucoadhesive surface 202 is arranged on or adjacent to, in particular aligned with or centered on the buccal mucosa when the insertable part 102 is inserted to said insertion depth. To achieve this, a distance l between the contact surface 106A at the elastic member/hinge 110, 112 and the retaining structure/cutout 114 (for example the circular end portion of the cutout 114, in which the mechanical connector 210 is to be retained) may be chosen accordingly. Said distance l may determine and in some examples correspond to (e.g., be equal to or substantially equal to) the distance at which the mucoadhesive surface 202 (e.g., a center thereof) is arranged from the contact surface 106A. The distance l may for example be between 2 cm and 4 cm, preferably between 2.5 cm and 3.5 cm for arranging the mucoadhesive surface 202 on the buccal mucosa.

In addition to (or instead of) the distance l, other aspects pertaining to the shape of the applicator 100 and/or the disposable part 200, in particular the physical dimensions thereof, may be specifically adapted to the human anatomy, in particular the portion of the nasal or oral mucosa that the mucoadhesive surface 202 is to be arranged on. For example, the insertable part 102, e.g., the distal clamping portion 102C thereof, may comprise a disk-shaped portion 102D, in which the cutout 114 is arranged. A lateral physical dimension wₐ of the disk-shaped portion 102D, for example its width and/or its diameter, may be adapted to the human buccal mucosa, e.g., such that the lateral physical dimension corresponds to a height of the buccal mucosa in vertical direction (i.e., toe-to-head of the patient). The lateral physical dimension, as used herein, may for example be measured parallel to the mucoadhesive surface 202 and/or perpendicular to an insertion direction for inserting the insertable part 102 into the oral cavity (e.g., a direction extending from the handle portion 102A to the clamping portion 102C, for example the horizontal direction in Figs. 5f, 5g). The lateral physical dimension wₐ of the disk-shaped portion 102D may for example be between 2.5 cm and 4 cm, preferably between 3.0 cm and 3.5 cm, in one example 3.2 cm. In some examples, the exterior part 104 may have a similar shape as the insertable part 102.

Additionally or alternatively, a lateral physical dimension w_{d} of the disposable part 200 (e.g., of the carrier 206) may be adapted to the to the human anatomy, e.g., the shape of the human buccal mucosa. The lateral physical dimension w_{d} of the disposable part 200 may for example be within the aforementioned ranges. In some examples, the lateral physical dimension w_{d} of the disposable part 200 may correspond to (e.g., be equal or substantially equal) to the lateral physical dimension wₐ of the disk-shaped portion 102D. In other examples, the disposable part 200 may be smaller than the disk-shaped portion 102D, e.g., to reduce an amount of material in the disposable part 200 for minimizing its environmental footprint.

In some examples, a radius of curvature of the insertable part 102 (e.g., of the lateral edge of the disk-shaped portion 102D) and/or of the disposable part (e.g., of the lateral edge of the carrier 206) may be adapted to the human anatomy, e.g., the shape of the human buccal mucosa. The radius of curvature of the lateral edges of one or both the disk-shaped portion 102D and the carrier 206 may for example be between 0.5 cm and 3 cm, in some examples between 1.25 cm and 2 cm, preferably between 1.5 cm and 1.75 cm.

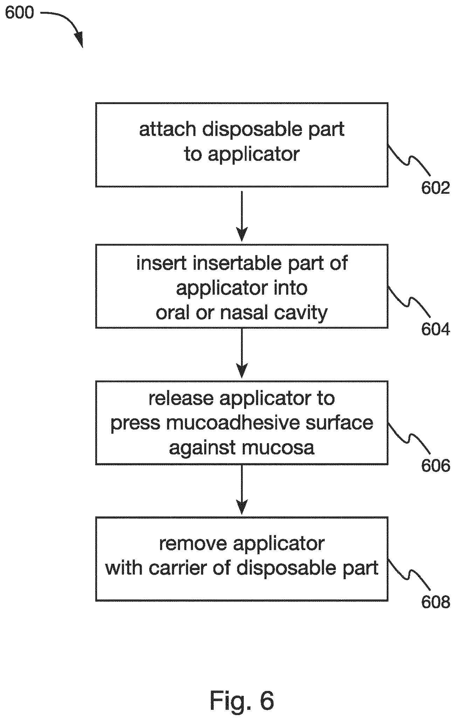

**Figure 6** shows a flow chart of a method 600 for arranging a mucoadhesive surface on the nasal or oral mucosa of a patient in accordance with an example. The method 600 may for example be used to arrange a mucoadhesive surface, in particular a mucoadhesive film loaded with an active agent, on the buccal mucosa of the patient, which is used as a non-limiting example of a portion of the nasal or oral mucosa of the patient for illustration purposes in the following. The method 600 may for example be executed with a kit, an applicator and/or a disposable part according to the present invention such as one of the kits 10, 20, 30, 40 and 50 described above, wherein the kit 50 is used as a non-limiting example for illustration purposes in the following. The method 600 is not limited to the order of execution implied by the flow chart in Fig. 6. As far as technically feasible, the method 600 may be executed in an arbitrary order and parts thereof may be executed simultaneously at least in part, for example steps 604 and 606 and/or steps 606 and 608.

In some examples, the applicator 100 and the disposable part 200 may be provided separately, i.e., with the disposable part 200 not being attached to or received by the applicator 100. In such cases, the method 600 may comprise, in step 602, attaching the disposable part 200 to the applicator 100, e.g., by inserting the mechanical connector 210 into the cutout 114 and snapping the mechanical connector 210 into the circular end portion of the cutout 114 to secure the disposable part 200 to the clamping portion 102C of the insertable part 102. The disposable part 200 is attached to the applicator 100 such that the mucoadhesive surface 202 faces the exterior part 104, in particular the clamping portion 104C thereof. In other examples, the applicator 100 may already be provided with the disposable part 200 attached thereto prior to execution of the method 600, in which case the method 600 may not comprise step 602.

With the disposable part 200 attached to the applicator 100, the insertable part 102, in particular the disk-shaped portion 102D and/or the clamping portion 102C thereof, is inserted into the oral (or nasal) cavity of the patient in step 604, e.g., until the contact surface 106A comes in contact with the corner of the mouth, at which point the insertable part 102 may be inserted at the predefined insertion depth. For inserting the insertable part 102 into the oral cavity, the handle portions 102A, 104A may be pressed together (e.g., to compress the elastic member 110) to open the applicator 100, i.e., to move the clamping portions 102C, 104C apart, e.g., to increase a separation distance therebetween. Step 604 further comprises placing the exterior part 104, in particular the clamping portion 104C thereof, on the skin of the patient (e.g., on the outside of the cheek) such that the cheek with the buccal mucosa is arranged between the interior and exterior parts 102, 104 (in particular the clamping portions 102C, 102D thereof) with the mucoadhesive surface 202 facing the buccal mucosa. In some examples, the mucoadhesive surface 202 may be brought in contact with the buccal mucosa in step 604.

The method 600 further comprises, in step 606, pressing the mucoadhesive surface 202 of the disposable part 200 against the buccal mucosa using the elastic member 112 of the applicator 100 (as an example of a means for pressing a mucoadhesive surface of a disposable part against the nasal or oral mucosa). This may for example comprise releasing the handle portions 102A, 104A such that the elastic member 112, which biases the clamping portions 102C, 104C towards each other, pushes the clamping portions 102C, 104C towards each other (closes the applicator 100), thereby bringing the mucoadhesive surface 202 in contact with the buccal mucosa (in case such contact was not already established in step 604 for example) and pressing the mucoadhesive surface 202 against the buccal mucosa. The mucoadhesive surface 202 may for example be pressed against the nasal or oral mucosa with a force of between 0.1 N and 50 N, preferably between 0.5 N and 10 N, most preferably between 2 N and 4 N, in some examples between 2.7 N and 3.3 N, e.g., 3.0 N. The mucoadhesive surface 202 may be pressed against the buccal mucosa for less than 10 min, preferably less than 5 min, most preferably less than 3 min. The mucoadhesive surface 202 may be pressed against the buccal mucosa for a time that is sufficient to achieve adhesion of the mucoadhesive surface 202 to the buccal mucosa, for example for between 0.5 s and 180 s, in some examples between 1 s and 120 s, in some examples between 10 s and 60 s, in one example between 20 s and 40 s. In other examples, the mucoadhesive surface 202 may be pressed against the buccal mucosa for a desired retention or application time of the mucoadhesive film 204 (e.g., for releasing a certain amount of the active agent and/or for dissolving the mucoadhesive film 204 or a part thereof), for example for between 10 s and 120 min, in some examples between 30 s and 60 min, in some examples between 1 min and 30 min and in some example between 30 min and 60 min.

The method 600 may further comprise removing the insertable part 102 of the applicator 100 from the oral (or nasal) cavity in step 608, e.g., by pressing the handle portions 102A, 104A together to open the applicator 100 again (i.e., move the clamping portions 102C, 104C apart) and moving the insertable part 102 out of the oral cavity. Preferably, the mucoadhesive surface 202 remains arranged on (e.g., continues to adhere to) the buccal mucosa when the insertable part 102 is removed. Accordingly, step 608 may comprise separating the mucoadhesive film 204 from the carrier 206 of the disposable part 200 while the mucoadhesive film 204 remains arranged on the buccal mucosa. This may in particular comprise overcoming a pull-off force of an adhesive such as the adhesive layer 204C attaching the mucoadhesive film 204 to the carrier 206 by an adhesion force between the mucoadhesive surface/film 202, 204 and the buccal mucosa, e.g., when opening the applicator 100 by pressing the handle portions 102A, 102A together. The mucoadhesive film 204 may thus stick to the buccal mucosa and separate from the carrier 206 at the adhesive layer 204C (e.g., by pulling the adhesive layer 204C off the carrier 206 and/or the mucoadhesive film) while the carrier 206 remains attached to the insertable part 102, e.g., via the mechanical connector 210 retained in the cutout 114 or via the (stronger) adhesive 208. After the insertable part 102 was removed from the oral cavity, the mucoadhesive film 204 may remain on the buccal mucosa for a desired retention or application time of the mucoadhesive film 204 (e.g., for releasing a certain amount of the active agent and/or for dissolving the mucoadhesive film 204 or a part thereof), for example for between 10 s and 120 min, in some examples between 30 s and 60 min, in some examples between 1 min and 30 min. Subsequently, the mucoadhesive film 204, unless already dissolved, may be removed from the oral cavity, e.g., by hand.

The features of the present invention disclosed in the specification, the claims, and/or in the accompanying figures may, both separately and in any combination thereof, be material for realizing the invention in various forms thereof.

## Claims

1. An applicator (100) for arranging a mucoadhesive surface (202), in particular a mucoadhesive film (204) loaded with an active agent, on the nasal or oral mucosa (12), in particular the buccal mucosa, of a patient, the applicator (100) comprising:
an insertable part (102) and an exterior part (104) connected to the insertable part (102), the insertable and exterior parts (102, 104) for insertion into the nasal or oral cavity of the patient and for placement on the skin (14) of the patient, respectively, with the nasal or oral mucosa (12) arranged therebetween, wherein
the insertable part (102) comprises a retaining means (114) that is configured to receive a disposable part (200) comprising the mucoadhesive surface (202) such that the mucoadhesive surface (202) faces the nasal or oral mucosa (12) when the nasal or oral mucosa (12) is arranged between the insertable part (102) and the exterior part (104); and
a means for pressing the mucoadhesive surface (202) against the nasal or oral mucosa (12) when the disposable part (200) is received by the retaining means (114) and the nasal or oral mucosa (12) is arranged between the insertable part (102) and the exterior part (104).

2. The applicator (100) of claim 1, wherein the means for pressing the mucoadhesive surface (202) against the nasal or oral mucosa (12) is or comprises an elastic member (102-106, 112), in particular a spring, configured to bias the disposable part (200), when received by the retaining means (114), or a part thereof towards the exterior part (104), in particular wherein:
the insertable part (102) and/or the exterior part (104) comprise(s) a different material than the elastic member (102-106,112); and/or
the elastic member (102-106,112) and one or both of the insertable part (102) and
the exterior part (104) are formed as a single piece, in particular by injection molding.

3. The applicator (100) of claim 1 or 2, wherein the means for pressing the mucoadhesive surface (202) against the nasal or oral mucosa (12) is configured to press the mucoadhesive surface (202) against the nasal or oral mucosa (12) with a force of between 0.1 N and 50 N, preferably between 0.5 N and 10 N, most preferably between 2 N and 4 N.

4. The applicator (100) of any one of the preceding claims, wherein the applicator (100) comprises a hinge (110) that pivotably connects the insertable part (102) and the exterior part (104), in particular wherein:
the insertable part (102) and the exterior part (104) each comprise a proximal handle portion (102A, 104A), a central hinge portion (102B, 104B) and a distal clamping portion (102C, 104C) with the retaining means (114) being arranged on and/or in the clamping portion (102C, 104C) of the insertable part (102), wherein the hinge (110) connects the hinge portions (102B, 104B) of the insertable and exterior parts (102, 104) so as to allow for moving the clamping portions (102C, 104C) apart from each other by pushing the handle portions (102A, 104A) towards each other; and/or
the applicator (100) comprises said elastic member (112) and wherein the hinge (110) is formed by or comprises the elastic member (112) or the elastic member (112) extends between the handle portions (102A, 104A) of the insertable and exterior parts (102, 104).

5. The applicator (100) of any one of the preceding claims, wherein the retaining means (114) is or comprises a retaining structure that is configured to removably receive the disposable part (200), in particular via a snap fit or a press fit.

6. The applicator (100) of any one of the preceding claims, wherein:
the applicator (100) further comprises a contact surface (106A) arranged between the insertable part (102) and the exterior part (104), wherein the contact surface (106A) is to come in contact with an edge (16) of the nasal or oral orifice of the patient, in particular a corner of the mouth, when the insertable part (102) is inserted into the nasal or oral cavity of the patient to a predefined insertion depth (d) to prevent further insertion of the insertable part (102) beyond said predefined insertion depth (d), in particular wherein a distance (1) between said contact surface and the retaining means (114) is between 0.5 cm and 6 cm, preferably between 2 cm and 4 cm, most preferably between 2.5 cm and 3.5 cm; and/or
the insertable part (102) comprises a disk-shaped portion (102D) in and/or on which the retaining means (114) is arranged, wherein preferably a lateral physical dimension (wₐ), in particular a diameter, of the disk-shaped portion (102D) is between 1.5 cm and 5 cm, most preferably between 2.5 cm and 4 cm.

7. A disposable part (200) for use with an applicator (100) for arranging a mucoadhesive surface (202) on the nasal or oral mucosa (12) of a patient, the applicator (100) comprising an insertable part (102) and an exterior part (104) connected to the insertable part (102), the insertable and exterior parts (102, 104) for insertion into the nasal or oral cavity of the patient and for placement on the skin (14) of the patient, respectively, with the nasal or oral mucosa (12) arranged therebetween, the disposable part (200) comprising:
a carrier (206) having a mucoadhesive surface (202); and
an attachment means (208, 210) for attaching the disposable part (200) to the insertable part (102) of the applicator (100) such that the mucoadhesive surface (202) of the disposable part (200) faces the nasal or oral mucosa (12) of the patient when the nasal or oral mucosa (12) is arranged between the insertable part (102) and the exterior part (104) of the applicator (100).

8. The disposable part (200) of claim 7, wherein the mucoadhesive surface (202) is arranged on a first side of the carrier (206) and the attachment means (208, 210) is arranged on a second side of the carrier (206) opposite to the first side.

9. The disposable part (200) of claim 7 or 8, wherein the disposable part (200) is for use with an applicator (100) according to any one of claims 1 to 6 and the attachment means (208, 210) is configured to be received by the retaining means (114) of the insertable part (102) of the applicator (100), wherein the attachment means (210) preferably is or comprises a mechanical connector that is configured to be removably received by the retaining means (114), in particular via a snap fit or a press fit.

10. The disposable part (200) of any one of claims 7 to 9, wherein the mucoadhesive surface (202) is formed by a mucoadhesive film (204) that is loaded with an active agent, in particular a drug, and arranged on and/or in the carrier (206), in particular wherein said mucoadhesive film (204) comprises a mucoadhesive polymeric matrix layer (204A) containing said active agent and, optionally, a backing layer (204B) that the mucoadhesive polymeric matrix layer (204A) is attached to.

11. The disposable part (200) of any one of claims 7 to 10, wherein the mucoadhesive surface (202) is removably attached to the carrier (206), in particular by an adhesive (204C), so as to allow for separating the mucoadhesive surface (202) from the carrier (206) when the mucoadhesive surface (202) is arranged on the nasal or oral mucosa (12).

12. A kit (10, 20, 30, 40, 50) comprising a disposable part (200) having a mucoadhesive surface (202) and an applicator (100) for arranging said mucoadhesive surface (202) on the nasal or oral mucosa (12) of a patient, wherein:
the applicator (100) comprises an insertable part (102) and an exterior part (104) connected to the insertable part (102), the insertable and exterior parts (102, 104) for insertion into the nasal or oral cavity of the patient and for placement on the skin (14) of the patient, respectively, with the nasal or oral mucosa (12) arranged therebetween;
the disposable part (200) is attached or attachable to the insertable part (102) of the applicator (100) such that the mucoadhesive surface (202) of the disposable part (200) faces the nasal or oral mucosa (12) when the nasal or oral mucosa (12) is arranged between the insertable part (102) and the exterior part (104) of the applicator (100); and
the applicator (100) comprises a means for pressing the mucoadhesive surface (202) of the disposable part (200) against the nasal or oral mucosa (12) when the disposable part (200) is attached to the insertable part (102) of the applicator (100) and the nasal or oral mucosa (12) is arranged between the insertable part (102) and the exterior part (104) of the applicator (100).

13. The kit (10, 20, 30, 40, 50) of claim 12, wherein the applicator (100) is an applicator (100) of any one of claims 1 to 6 and/or the disposable part (200) is a disposable part (200) of any one of claims 7 to 11.

14. The kit (10, 20, 30, 40, 50) of claim 12 or 13, wherein the means for pressing the mucoadhesive surface (202) against the nasal or oral mucosa (12) are configured to apply a pressure of between 0.1 kPa and 1000 kPa, preferably between 2.5 kPa and 100 kPa, most preferably between 4 kPa and 50 kPa, between the mucoadhesive surface (202) of said disposable part (200) and the nasal or oral mucosa (12).

15. The kit (10, 20, 30, 40, 50) of any one of claims 12 to 14, wherein the mucoadhesive surface (202) is formed by a mucoadhesive film (204) removably attached to the disposable part (200) so as to allow for separating the mucoadhesive film (204) from the disposable part (200) when the mucoadhesive film (204) is arranged on the nasal or oral mucosa (12), in particular wherein the mucoadhesive film (204) is removably attached to the disposable part (200) by an adhesive, preferably an adhesive layer(204C), having a pull-off force that is lower than a pull-off force for separating the mucoadhesive film (204) from the nasal or oral mucosa (12) after the mucoadhesive film (204) was pressed against the nasal or oral mucosa (12) by said means for pressing the mucoadhesive surface (202) of the disposable part (200) against the nasal or oral mucosa (12).
